# EUROPÄISCHE PATENTANMELDUNG

(11) **EP 1 777 292 A1**
(43) Veröffentlichungstag der Anmeldung: **25.04.2007**
(21) Anmeldenummer: 05022789.1
(22) Anmeldetag: 19.10.2005
(51) Int. Cl.: C12N 15/11

(54) **Verfahren zum Erzeugen von genetischer Diversität in vivo**

(71) Anmelder: Signalomics GmbH, 48565 Steinfurt (DE)
(72) Erfinder: Block, Christoph, Dr., 48151 Münster (DE); Arntz, Claudia, Dr., 49525 Lengerich (DE)
(74) Vertreter: Von Renesse, Dorothea

(57) **Zusammenfassung**

Verfahren zur Erzeugung von genetischer Diversität *in vivo* umfassend folgende Schritte:
- Erzeugen von Fragmenten mindestens einer Wildtyp-Gensequenz, wobei die Fragmente jeweils mindestens einen zu einem für die *in vivo* Rekombination geeigneten Vektor homologen Bereich aufweisen;
- Erzeugen von randomisierten Überbrückungsoligonukleotiden mindestens einer definierten Oligonukleotidseqenz, wobei Teile des Überbrückungsoligonukleotids mit mindestens einem Fragment der Wildtyp-Gensequenz homolog sind und
- Einbringen des linearisierten Vektors, mindestens eines Wildtyp-Fragments sowie des/der randomisierten Überbrückungsoligonukleotide in ein *in vivo* System zur homologen Rekombination.

SEQUENCE LISTING

<110>  Signalomics GmbH

<120>  Verfahren zum Erzeugen von genetischer Diversität in vivo

<130>  46 779 K

<140>  EP 05 022 789.1
<141>  2005-10-19

<160>  35

<170>  PatentIn version 3.3

<210>  1
<211>  37
<212>  DNA
<213>  artificial

<220>
<223>  Primer QC-RafRBD-BstBI

<400>  1
aagaacagtg gtcaatgttc gaaatggaat gagcttg                                37

<210>  2
<211>  48
<212>  DNA
<213>  artificial

<220>
<223>  Primer QC-RafRBD-AscI

<400>  2
acgaacacaa aggtaaaaag gcgcgcctag attggaatac tgatgctg                    48

<210>  3
<211>  28
<212>  DNA
<213>  artificial

<220>
<223>  Primer FPO-out-for

<400>  3
actatctatt cgatgatgaa gatacccc                                          28

<210>  4
<211>  23
<212>  DNA
<213>  artificial

<220>
<223>  Primer FPO-out-rev

<400>  4
tggcggccgt tacttactta gag                                               23

<210>  5
<211>  26
<212>  DNA
<213>  artificial

<220>
<223>  Primer FPO-in-rev

<400>  5
ataaggcagt catgcaagct cattcc                                          26


<210>  6
<211>  20
<212>  DNA
<213>  artificial

<220>
<223>  Primer FPO-in-for

<400>  6
aggggcctgc aaccagagtg                                                 20


<210>  7
<211>  62
<212>  DNA
<213>  artificial

<220>
<223>  Primer FPO-NNK


<220>
<221>  misc_feature
<222>  (31)..(32)
<223>  n is a, c, g, or t

<400>  7
ggaatgagct tgcatgactg ccttatgaaa nnkctcaagg tgaggggcct gcaaccagag      60

tg                                                                    62


<210>  8
<211>  116
<212>  DNA
<213>  artificial

<220>
<223>  Primer FPO-NNK 3


<220>
<221>  misc_feature
<222>  (55)..(56)
<223>  n is a, c, g, or t

<400>  8
agaacagtgg tcaatgttcg aaatggaatg agcttgcatg actgccttat gaaannkctc      60

aaggtgaggg gcctgcaacc agagtgctgt gcagtgttca gacttctcca cgaaca         116


<210>  9
<211>  31
<212>  DNA
<213>  artificial

<220>
<223>  Primer 365-for

<400>  9

acgggtaccg caaagggaag ggatgctaag g 31

<210> 10
<211> 30
<212> DNA
<213> artificial

<220>
<223> Primer 394-for

<400> 10
atcggtacct gaacgttaca gaaaagcagg 30

<210> 11
<211> 31
<212> DNA
<213> artificial

<220>
<223> Primer 1563-rev

<400> 11
actactagtg cctcttcgct attacgccag c 31

<210> 12
<211> 27
<212> DNA
<213> artificial

<220>
<223> Primer 1674-rev

<400> 12
agaactagtg gaagatcgca ctccagc 27

<210> 13
<211> 31
<212> DNA
<213> artificial

<220>
<223> Primer 1451-rev

<400> 13
acaactagta acttttcggc caatggtctt g 31

<210> 14
<211> 37
<212> DNA
<213> artificial

<220>
<223> Primer 1366-rev

<400> 14
actactagtc ctatagtttt ttctccttga cgttaaa 37

<210> 15
<211> 34
<212> DNA
<213> artificial

<220>
<223>  Primer RedStar-for

<400>  15
actactagtt atgagtagat cttctaagaa cgtc                                34


<210>  16
<211>  33
<212>  DNA
<213>  artificial

<220>
<223>  Primer RedStar-rev

<400>  16
tattccgcgg ttacaagaac aagtggtgtc tac                                 33


<210>  17
<211>  781
<212>  DNA
<213>  artificial

<220>
<223>  Sequence cobA (orininally stemming from Propionibacterium
       freudenreichii) optimized for the frequentness of codon usage in
       S. cerevisiae

<400>  17
tcatgaccac tactttattg ccaggtactg ttactttggt tggtgcaggt ccaggtgatc     60

cagaattggt tacagttgct ggtttaagag cagttcaaca agctgaagtt attctatacg    120

atagattggc tccacaagat ttgttgtctg aagcatctga cgatgcagaa ttagttccag    180

ttggtaaaat tccaagagga cattacgttc cacaggagga aattaatcaa ttattggttg    240

ctcatgctag agaaggtaga aaagtagtta gattaaaggg tggagattct ttcgtttttg    300

gtagaggtgg agaggaatgg caagcatgtg ctgaagctgg tattccagtt agagttattc    360

caggagtttc ttctgctact gctggtccag ctttggctgg tattcctttg actcatagac    420

atttggttca aggttttact gttgtttcag gtcatgtttc tccatcagat gaaagatcag    480

aagttccttg gagacaattg gctaaggata gattgacttt ggttatttta atgggagttg    540

ctcatatgag agatattgct ccagaattga tggctggagg tttaccagca gatactcctg    600

ttagagtagt ttctaatgct tctttggctt ctcaggaatc ttggagaact actttgggag    660

atgcagttgc tgatatggat gctcatcacg ttagaccacc tgctttggta gtagttggta    720

ctttggctgg agtagatttg tctcatccag atcatagagc accatcagat cataactcga    780

g                                                                    781


<210>  18
<211>  32
<212>  DNA
<213>  artificial

<220>
<223>  Primer Met1-for

<400>  18

aattatccat ggtacgagac ttagtgacat tg 32

<210> 19
<211> 45
<212> DNA
<213> artificial

<220>
<223> Primer Met1-rev

<400> 19
aattaactcg agttgtataa cttaaataga ctatctacat caacc 45

<210> 20
<211> 36
<212> DNA
<213> artificial

<220>
<223> Primer KEX2-for

<400> 20
atccttgagc tctcagcagc tctgatgtag atacac 36

<210> 21
<211> 43
<212> DNA
<213> artificial

<220>
<223> Primer KEX2-rev

<400> 21
atcccccatg gctgataatg ggttagtagt ttataattat gtg 43

<210> 22
<211> 33
<212> DNA
<213> artificial

<220>
<223> Primer TEF-for

<400> 22
atccccgcgg tagcttcaaa atgtttctac tcc 33

<210> 23
<211> 38
<212> DNA
<213> artificial

<220>
<223> Primer TEF-rev

<400> 23
atcccccatg gtttgtaatt aaaacttaga ttagattg 38

<210> 24
<211> 36
<212> DNA
<213> artificial

<220>
<223>  Primer GAPDH-for

<400>  24
atccccgcgg cagttcgagt ttatcattat caatac                                36


<210>  25
<211>  36
<212>  DNA
<213>  artificial

<220>
<223>  Primer GAPDH-rev

<400>  25
atcccccatg gtttgtttgt ttatgtgtgt ttattc                                36


<210>  26
<211>  45
<212>  DNA
<213>  artificial

<220>
<223>  Primer Term-Raf-for

<400>  26
ctatataact ctgtagaaat aaagagtatc atctttcaaa gagct                      45


<210>  27
<211>  45
<212>  DNA
<213>  artificial

<220>
<223>  Primer Term-Raf-rev

<400>  27
ctttgaaaga tgatactctt tatttctaca gagttatata gacgt                      45


<210>  28
<211>  32
<212>  DNA
<213>  artificial

<220>
<223>  Primer RafRBD-Pci-for

<400>  28
aattccacat gtccgacccg agtaagacaa gc                                    32


<210>  29
<211>  40
<212>  DNA
<213>  artificial

<220>
<223>  Primer RafRBD-SacII-rev

<400>  29
attgccgcgg ttagtcgaca tctagaaaat ctacttgaag                            40


<210>  30

<211>  50
<212>  DNA
<213>  artificial

<220>
<223>  Primer Multi-QC-Pme-vor-TEM

<400>  30
tgaatactca tactcttcct gtttaaacat tattgaagca tttatcaggg                50


<210>  31
<211>  52
<212>  DNA
<213>  artificial

<220>
<223>  Primer Multi-QC-Pme-nach-TEM

<400>  31
ttaaatcaat ctaaagtata tatgtttaaa cttggtctga cagttaccaa tg             52


<210>  32
<211>  47
<212>  DNA
<213>  artificial

<220>
<223>  Primer Pme-Kan-for

<400>  32
aaaaaaccgt ttaaacagga agagtatgat tgaacaagat ggattgc                   47


<210>  33
<211>  50
<212>  DNA
<213>  artificial

<220>
<223>  Primer Pme-Kan-rev

<400>  33
aaaaaaccgt ttaaacttgg tctgacagtc agaagaactc gtcaagaagg                50


<210>  34
<211>  41
<212>  DNA
<213>  artificial

<220>
<223>  Primer Z-RBD-A85-for1


<220>
<221>  misc_feature
<222>  (14)..(15)
<223>  n is a, c, g, or t

<400>  34
ctgccttatg aaannkctca aggtgagggg cctgcaacca g                         41


<210>  35
<211>  41
<212>  DNA

<213>  artificial

<220>
<223>  Primer Z-RBD-A85-rev


<220>
<221>  misc_feature
<222>  (15)..(16)
<223>  n is a, c, g, or t

<400>  35
ccctcacctt gagmnntttc ataaggcagt catgcaagct c                41

## Beschreibung

Die Erfindung betrifft ein einfaches und effizientes Verfahren zum Erzeugen von genetischer Diversität *in vivo*, insbesondere zum Erzeugen repräsentativer DNA-Banken.

Das Erzeugen genetischer Mutanten als Ausgangsbasis für das Auffinden neuartiger Proteine mit verbesserten Eigenschaften ist von aktueller Bedeutung. Dazu sind nicht-rekombinative und rekombinative Methoden der Mutagenese bekannt. Während beispielsweise die error prone PCR (epPCR) und saturation mutagenesis (SM) zu den nicht-rekombinativen Verfahren gezählt werden, gehören alle shuffling-Ansätze zu den rekombinativen Methoden. Selbst die shuffling-Ansätze greifen jedoch häufig zum Erzeugen der Diversität auf PCR-Verfahren zurück.

Muhlrad et al. (1992) haben z.B. das Gen für den a Faktor des Mating Pheromons (MFA2) aus Hefe mittels einer epPCR mutagenisiert. Aus diesen Ergebnissen werden die Nachteile der epPCR deutlich. Die epPCR als solche beruht nämlich entweder auf einer Einfügung einer falschen Base und/oder auf dem Fehlen der Korrekturlesefähigkeit der Polymerase. Die inhärente Eigenschaft der verwendeten Polymerase bedeutet jedoch, dass einige Fehler häufiger auftreten als andere. Damit liegen dann einige Mutationen (wie z.B. Transitionen) häufiger vor als andere und die Bank hat eine nicht-zufällige Beschaffenheit ("bias"). Dies führt zu einem sog. error bias und demnach zu einer fehlenden Repräsentativität der erzeugten DNA-Bank. Bei Muhlrad et al. (1992) stehen beispielsweise 32 Transitionen, von denen 31 A/T zu G/C sind, nur 7 Transversionen gegenüber, unter denen G/C zu C/G gar nicht beobachtet wurde.

Weiterhin tritt bei PCR-basierten Verfahren der sogenannte "codon bias" auf, der auf der Natur des genetischen Codes beruht. Einfache Punktmutationen führen zu einem bias an Aminosäuren, die das mutierte Codon codiert. Zum Beispiel kann eine Punktmutation in einem Valin-Codon nur zu sechs anderen Aminosäuren führen, nämlich zu Phe, Leu, Ile, Ala, Asp oder Gly. Zur Codierung der anderen Aminosäuren sind hingegen entweder zwei (C, S, P, H, R, N, T, M, E, Y) oder sogar drei Punktmutationen (Q, W, K) erforderlich.

Ein Nachteil der epPCR ist auch, dass nicht alle Basen für eine Mutagenisierung zugänglich sind und dass, statistisch gesehen, eine gegebene Aminosäure nur zu ca. fünf anderen Aminosäuren mutagenisiert wird.

Ein weiterer "bias", der bei jeder PCR auftritt, ist der "amplification bias". Ein Molekül, das früh im Amplifizierungsprozess kopiert wurde, ist in der endgültigen Bank bzw. dem PCR-Ansatz überrepräsentiert. Dieses kann dazu führen, dass eine bestimmte Mutante demgegenüber in so geringer Anzahl vertreten ist, dass sie in einem Screening kaum detektiert werden kann. Dieses Problem kann durch eine Kombination von verschiedenen getrennt durchgeführten (ep)PCRs und/oder durch eine Reduktion der PCR-Zyklenanzahl nur teilweise überwunden werden.

Auch andere Methoden der Mutagenese, beispielsweise die sog. "sequence saturation mutagenesis" oder die RID-Methode ("random insertion and deletion") setzen die PCR ein, um genetische Vielfalt zu erzeugen. Damit weisen auch die damit erzeugten Banken ein Ungleichgewicht - d.h. eine fehlende Zufälligkeit oder Repräsentativität - zwischen den erzeugten Varianten auf. Darüber hinaus sind diese Methoden kompliziert und aufwändig.

Im Gegensatz zu den oben genannten Methoden, bei denen eine längere DNA-Sequenz nur teilweise und zufällig mutagenisiert wird, werden bei der saturation mutagenesis (SM) Varianten der DNA-Sequenz erzeugt, in denen jede Aminosäure durch alle anderen natürlich vorkommenden Aminosäuren ersetzt wird. Bei dieser Methode ist die Anzahl der entstehenden Varianten naturgemäß abhängig von der Länge der eingesetzten Sequenz. Es entstehen sehr viele Varianten, die alle auf ihre Eigenschaften hin, wie z.B. auf ihre Sequenz, überprüft werden müssen.

Für den Austausch einer Aminosäure gegen Jede andere oder zum Einbringen von anderen gezielten Mutationen ist der Einsatz von degenerierten Oligonukleotiden eine geeignete Methode, die vielfältig variiert und weiterentwickelt wurde. Dabei können eine (oder mehrere) synthetische DNA-Sequenzen (Oligonukleotide/Inserts), die unterschiedlich stark mutagenisiert sein können, in die codierende Sequenz inkorporiert werden. Der weitaus größte Teil dieser Verfahren verwendet jedoch ebenso die PCR-Technologie wenigstens zur Amplifikation von Teilen der erzeugten genetischen Varianten und ist somit mit den oben geschilderten Nachteilen behaftet.

Bei dem sog. "Gene Assembly" wird ein Gen aus mehreren kleineren Einheiten ("Oligonukleotiden") zusammengefügt. Dieses Verfahren kann bewusst zum Erzeugen von Diversität genutzt werden; indem ein Wildtyp-Oligonukleotid durch ein degeneriertes Oligonukleotid ersetzt wird. Damit wird in dem resultierenden Gen eine Mutation eingebaut. Ein solches Verfahren ist von Stemmer et al. (1994) bekannt. Dabei wird jedoch die Amplifikation des Gens wiederum mittels PCR - und daher mit dem Nachteil des "amplification bias" - durchgeführt.

Das ebenso bekannte shuffling zählt zu den rekombinativen Methoden der evolutiven Mutagenese. Dabei werden Fragmente, bei denen es sich beispielsweise um Restriktionsfragmente handeln kann, neu zusammengefügt. Da es sich um homologe Fragmente unterschiedlicher Herkunft (verschiedene Allele, verschiedene Isoformen eines Enzyms, verschiedene Punktmutationen usw.) handelt, entstehen dabei neue genetische Varianten mit veränderten (möglichst verbesserten) Eigenschaften.

Bei dem DNA-shuffling wird die fragliche Sequenz mit DNasel verdaut, die Fragmente werden in einer primerlosen PCR zusammengefügt und das Produkt dieser Reaktion wird mittels einer PCR amplifiziert. Auch andere Varianten des "shufflings" setzen eine PCR ein und weisen daher wiederum die oben geschilderten Nachteile, insbesondere den amplification bias, auf. Um dennoch alle möglichen Mutanten zu erfassen, muß die Anzahl der gescreenten Transformanten deutlich erhöht werden. Dies ist jedoch in der Praxis kaum umsetzbar.

Eine erfolgreiche Mutagenese setzt jedoch nicht nur das Erzeugen von repräsentativen Mutanten (eine "ausgeglichene" und "zufällige" Diversität), sondern auch die anschließende Transformation eines Wirtsorganismus zur Expression der codierten Proteine voraus. Dabei greift der Großteil der bekannten Methoden auf die Ligation (z.T. Restriktion) und Klonierung der bei der Mutagenese erhaltenen Bankfragmente In einen Vektor zurück.

Dieser Weg ist jedoch generell zeitaufwändig. Die Diversität der resultierenden und exprimierten DNA-Bank ist abhängig von einer effizienten Ligation und Bakterientransformation. Beides sind jedoch Schritte, die für Störungen anfällig sind und zu einer Vorselektion der Mutageneseprodukte führen können. Eine geringe Effizienz einer dieser beiden Schritte hat eine zu geringe Größe der Bank zur Folge. Die resultierende Bank ist dementsprechend nicht repräsentativ, da sie nicht die erforderliche Menge an unabhängigen Klonen beinhaltet. Dies ist vor allem dann bedeutsam, wenn an mehr als einer Position randomisiert (mutagenisiert) wird. Beispielsweise müssen bei zwei mutagenisierten Codons ca. 3100, bei drei Codons bereits 10⁵ Klone gescreent werden, um mit 95%iger Wahrscheinlichkeit die am geringsten repräsentierte Doppel- bzw. Tripelmutante zu finden (Hogrefe et al. 2002).

Palfrey et al. (2000) beschreiben den Effekt des Klonierens auf die Verteilung der Mutanten in einer Bank. Sie zeigen deutlich, dass die Verteilung der Oligonukleotidsequenzen, die in den Vektor pUC19 ligiert werden, nicht in der Verteilung der Plasmidsequenzen nach der Klonierung wiederzufinden ist. Dabei scheint dieser Effekt nicht auf einem selektiven Druck zu beruhen, da verschiedene Sequenzen in unterschiedlichen Banken favorisiert werden.

Außerdem muss für den Einsatz der Bank in anderen Organismen Plasmld-DNA extrahiert werden. Dieses bedeutet Wachstum der Bakterien, die die Bank beherbergen. Dieser Prozess, der wahrscheinlich auf den möglichen unterschiedlichen Wachstumseigenschaften der Individuellen Klone beruht, kann den Verlust der Komplexität der Bank bedeuten.

Bosley und Ostermeier (2005) entwickelten mathematische Ansätze für die Konstruktion, Beschreibung und Evaluation von Banken. Sie weisen darauf hin, dass nach einer Subklonierung und/oder Retransformation die Anzahl der Transformanten sehr viel größer sein muss, als die Anzahl der Transformanten in der Ursprungs-Bank, um die Anzahl der unabhängigen Klone der Original-Bank aufrecht zu erhalten.

Es ist bekannt, dass ein Teil der genannten Probleme durch den Einsatz der homologen Rekombination vermieden werden kann. Diese ist u.a. auch von der Hefe als sog. "gap repair"-Mechanismus bekannt. Dabei rekombinieren freie DNA-Enden mit der homologen Region in einem Hefechromosom. Das Produkt ist zirkulär und enthält die Sequenz des Chromosoms zwischen den rekombinogenen Enden. Verschiedene Arbeitsgruppen haben diese homologe Rekombination für die Konstruktion von Plasmiden und das *in vivo* Klonieren eingesetzt. Dabei werden ein geschnittener Vektor und ein Insert, das an den Enden Homologie zu den Enden des Vektors aufweist, in die Hefezellen kotransformiert.

Es ist bekannt, die homologe Rekombination neben ihrem Einsatz zur Klonierung von Plasmiden *in vivo* (s.o.), auch zur Generierung von Banken *in vivo* einzusetzen. So haben Hua et al. (1998) eine Two-hybrid cDNA-Bank von humanen EST-Klonen In einem Gal4 AD-Vektor durch homologe Rekombination erzeugt. Auch Fusco et al. (1999) klonierten eine cDNA Bank *in vivo.* Schaerer-Brodbeck und Barberis (2004) haben durch Replacement der CDR3 (complementarity determining region) einer variablen Domäne der leichten Kette eines Antikörpers durch homologe Rekombination in Hefe eine Bank generiert. Das Insert wurde jedoch über PCR erhalten, mit dem Nachteil des amplification bias (s.o.). Das gleiche gilt im Ergebnis für andere Methoden, die auch jeweils eine PCR und/oder epPCR einsetzen (z.B. Muhlrad et al. 1992).

Zusammenfassend ist zu sagen, dass bei allen bekannten Methoden zur Mutagenese die Erzeugung und/oder die Amplifizierung der Bank-Fragmente durch PCR limitierend im Bezug auf die Diversität und Repräsentativität der Bank ist. Oft kommen noch die Nachteile der Klonierung mit den Schritten der Ligation und Transformation hinzu, die ebenfalls diese Eigenschaften der Bank beeinträchtigen. Außerdem sind viele Methoden kompliziert und/oder kosten- und/oder zeitaufwändig.

Aufgabe der Erfindung ist daher die Bereitstellung einer Bank, die ein Höchstmaß an repräsentativer Diversität in den ausgewählten Bereichen erlaubt und gleichzeitig möglichst schnell und einfach zu generieren ist. Der Begriff "Repräsentativität" wird im vorliegenden Kontext als Terminus für eine möglichst hohe Ausgeglichenheit (fehlender "bias") und hohe Zufälligkeit der Varianten gebraucht.

Die Erfindung löst diese Aufgabe durch die (Ko-)Transformation eines zur homologen Rekombination fähigen Wirtsorganismus mit Wildtyp-Fragmenten eines ausgewählten Gens, randomisierten Oligonukleotiden ("randomisierten Überbrückungsoligonukleotiden") und einem Vektor. Um die Rekombination zwischen diesen DNA-Abschnitten zu ermöglichen, weisen die Wildtyp-Fragmente an ihren Enden jeweils einen zu bestimmten Vektorabschnitten homologen Bereich auf. Ebenso weisen auch die Wildtyp-Fragmente einen homologen Bereich zu dem randomisierten Überbrückungsoligonukleotid auf.

Randomisiert im Sinne der Erfindung bedeutet, dass Basen eines Oligonukleotids in einer Population von Oligonukleotiden substituiert werden. Dabei kann ein zu dieser Population gehöriges Oligonukleotid nur eine oder aber auch mehrere Basensubstitutionen (z.B. im Rahmen eines Codons) aufweisen. Vorzugsweise umfasst jedes Oligonukleotid im Vergleich zur Wildtypsequenz mindestens eine Basensubstitution und innerhalb der Population ist jede Base mit allen denkbaren Basen substituiert. Demnach stellt ein "randomisiertes (Überbrückungs)Oligonukleotid" im Sinne der Erfindung möglichst die Summe aller theoretisch denkbaren Oligonukleotidvarianten der Wildtypsequenz bezogen auf die zu randomisierenden Position(en) dar. Falls alle denkbaren Varianten erfaßt wären, wäre die Randomisierung der Mutationen vollständig bzw. "saturiert".

Die Substitution kann auch nicht-natürliche Basen oder modifizierte Basen umfassen. Die Oligonukleotide können ein oder aber auch mehrere Codons umfassen. Der Begriff "randomisiertes Insert" wird als Synonym zu "randomisiertem Oigonukleotid" verwendet.

Der Begriff "Überbrückungsoligonukleotid" wurde gewählt, um deutlich zu machen, dass das Oligonukleotid ein Verbindungsglied zwischen den fragmentierten Wildtyp-sequenzen darstellt.

Durch dieses Vorgehen wird innerhalb des Wirtsorganismus ein "Gene Assembly" bei gleichzeitiger Inkorporation eines mutagenisierten Abschnitts - nämlich dem randomisierten Oligonukleotid - erreicht. Der Einsatz der homologen Rekombination erlaubt den Verzicht auf die - im Sinne der Erfindung - nachteiligen Klonierungsschritte.

Erfindungsgemäß stellt lediglich das randomisierte Überbrückungsoligonukleotid die Diversität der erzeugten DNA-Bank sicher. So stellt dieser Bereich den einzigen Abschnitt dar, der von der Wildtyp-Sequenz abweicht. Um die Repräsentativität der Bank zu gewährleisten, wird die Randomisierung/Mutagenese jedoch nicht über eine PCR sondern über nicht-PCR basierte Verfahren erzeugt. Ebenso wird erfindungsgemäß auf eine Amplifikation dieser Fragmente durch eine PCR verzichtet. Die Mutagenese-Produkte werden außerdem ohne Zwischenklonierung in den Zielorganismus transformiert. Da die homologe Rekombination nicht mutagen ist, bleibt die Komplexität und Diversität der Bank somit erhalten.

Vorzugsweise werden die randomisierten Überbrückungsoligonukleotide synthetisch hergestellt. Auch die Wildtypfragmente können synthetisch erzeugt werden.

Im Gegensatz dazu ist es durchaus möglich, die Wildtyp-Fragmente mittels eines PCR-basierten Verfahrens zu generieren. Da mit der Fragmentierung der Wildtyp-Sequenz allerdings keine Diversität erzeugt werden soll, werden keine degenerierten Primer verwendet, so dass durch die Verwendung einer PCR für diese Fragmente keine Unausgewogenheit der generierten DNA-Bank zu befürchten ist. Ebenso wenig enthalten die gesamten Überlappungsbereiche zu der Vektorsequenz, oder aber zu den randomisierten Inserts, mutagenisierte Bereiche.

Um die erzeugte Bank auf eine in der Praxis handhabbare Größe zu begrenzen, ist es vorteilhaft, lediglich ein oder nur wenige Codons eines Oligonukleotids zu randomisieren. Die ausgewählten Oligonukleotide sollten zudem möglichst kurz sein. Diese Randomisierung sollte dann jedoch auch "gesättigt" ("saturated"), d.h. möglichst vollständig, sein, so daß insgesamt ein akzeptables Verhältnis zwischen Bankgröße und dem späteren Screeningaufwand sichergestellt ist.

Die homologe Rekombination kann in Wirtsorganismen stattfinden, die ein funktionelles Rekombinationssystem besitzen. In Frage kommen außer Hefe *(S. cerevisiae,* andere Hefen wie z.B. *Schizosacchsromyces pombe)* auch Bakterien (z.B. Ba*cillus subtilis, E.coli*), Protozoen (Plasmodium, Toxoplasma), filamentöse Pilze (z.B. *Ashbya gossypii*), Pflanzen oder tierische Zellen (z.B. Säugerzellen oder Huhn DT40 Zellen).

Die homologe Rekombination in dem Wirtsorganismus (z.B. der Hefe, bei der Transformationsraten von 2 x 10⁴ bis 3 x 10⁶ Transformanten/µg DNA erzielt werden können) kann durch verschiedene Bedingungen beeinflusst werden, wie vor allem der Länge, dem Design und der Anzahl der rekombinogenen Enden sowie dem molaren Verhältnis der Vektor-DNA zu Insert-DNA (Wildtyp-Fragmente und DNA des Überbrückungsoligonukleotids), das zur Transformation eingesetzt wird. Diese Parameter können sich auch gegenseitig beeinflussen.

So können beispielsweise bis zu 6 Fragmente gemeinsam mit dem geschnittenen Vektor erfolgreich kotransformiert werden. Dazu können z.B. 10 ng eines jeden Wildtyp(PCR)-Fragments und z.B. 200 ng des linearisierten Vektors eingesetzt werden. Diese Angaben haben sich vor allem bei der Hefetransformation als möglich und sinnvoll erwiesen.

Ebenso ist es z.B. möglich, 100 ng Vektor-DNA mit 40 x molarem Überschuss des Inserts oder aber 200 ng Vektor-DNA mit 5 x molarem Überschuss an Insert-DNA zu verwenden. Es können aber auch 100 ng Vektor mit 1 µg Insert-DNA oder bis zu 100 ng Vektor mit 2 µg Insert-DNA plus 1 µg an 4 einzelsträngigen Oligonukleotiden eingesetzt werden. Insgesamt hat sich gezeigt, dass das molare Verhältnis zwischen geschnittenem Vektor und Insert-DNA vorzugsweise zwischen 1:1 bis 1:50, besonders bevorzugt zwischen 1:1 und 1:40 liegt. Eine Optimierung ist für jedes System angebracht.

Es hat sich gezeigt, dass eine Überlappung der rekombinogenen Enden der Fragmente von mindestens jeweils 40 bp zu einem besonders hohen Anteil positiver Klone, nämlich 90 %, führt. Die Verlängerung der Überlappungsbereiche auf 80 bp an beiden Enden kann bis zu 98 % positiver Kolonien führen. Die Überlappung von 60 bp an beiden Enden reichen aber bereits für 95 % aus. Auch ein Überlappungsbereich von nur 15 bp ergibt jedoch eine ausreichende Anzahl positiver Klone.

Erfindungsgemäß sollte das randomisierte Oligonukleotid daher lang genug sein, um eine Rekombination mit dem/den Wildtyp-Fragmenten und dem Vektor zu ermöglichen. Die Überlappungsbereiche umfassen vorzugsweise mindestens 10, 20, 30, 40, 50 oder 60 bp. Damit sollte die Länge der Oligonukleotide mindestens 23, 43, 63, 83, 103 oder 123 bp betragen, wenn nur ein Condon degeneriert werden soll. Wählt man die Überlappungsbereiche auf beiden Seiten des randomisierten Oligonukleotids unterschiedlich lang, kann das Oligonukleotid auch Längen annehmen, die zwischen diesen angegebenen Zahlen liegen.

Die Wildtypfragmente können verschiedene Längen annehmen, die der Größe des Gens und der Position/Länge des/der randomisierten Überbrückungsoligonukleotids angepasst sind.

Vorteilhafterweise wird eine vollständige Randomisierung an ausgewählten Positionen/Sequenzabschnitten der zu mutierenden Wildtypgensequenz durchgeführt. Zur Wahl dieser Position bzw. des Abschnitts sollten möglichst viele Kenntnisse über deren Funktion vorhanden sein. Liegt die Aminosäure(n) bzw. der Bereich fest, können die Primer für die Erzeugung der Wildtyp-Fragmente so gewählt werden, dass die Wildtyp-Fragmente den entsprechenden Bereich umschließen, oder - sofern nur ein Wildtypfragment verwendet wird - an dem zu mutagenisierenden Bereich angrenzen. Die Festlegung der Wildtyp-Fragmente sollte daher erfindungsgemäß mit der Lage und Position des zu mutagenisierenden DNA-Sequenzabschnitts korrespondieren.

In einer vorteilhaften Ausführungsform wird das erfindungsgemäße Verfahren in Hefe als Wirtsorganismus durchgeführt und mit einem Two-Hybrid System oder Varianten bzw. Weiterentwicklungen davon zur Evaluierung der Interaktion zweier Bindungsproteine kombiniert. In einem solchen System findet eine doppelte Selektion statt: Es können nur Hefekolonien wachsen, die eine Rekombination erfahren haben, da die Rekombination in einem Gen für einen Bindungspartner, und damit in einem für das selektive Wachstum notwendigen Gen erfolgt ist; es müssen alle Fragmente eingebaut worden sein. Hier wird also nicht nur, wie in herkömmlichen Systemen, auf das Vorhandensein des zirkulären Vektors selektiert, sondern zusätzlich auf das Vorhandensein und die Funktionalität des rekombinierten Gens. Es liegt also bei den erhaltenen Transformanten eine Mutationseffizienz von 100 % vor.

Bei der Verwendung eines Two-Hybrid Systems oder einer Weiterentwicklung davon ist es vorteilhaft, dass im Vektor gelegene, zur homologen Rekombination verwendete Homologiebereiche nicht zwischen den beiden eingesetzten Vektoren identisch sind, da es sonst zur Rekombination zwischen dem mutagenisierten Fragment und dem zweiten Vektor in der Zelle kommen kann.

Außerdem ist diese Erfindung schnell, einfach und variabel, da durch die Verwendung von Wildtyp-Fragmenten und randomisierten Oligonukleotiden und deren anschließender homologer Rekombination nicht in jedem Fall Restriktionsschnittstellen im Gen vorhanden sein müssen. Die Variation der Oligonukleotidsequenz, beispielsweise über synthetische Verfahren, und der angrenzenden Fragmente, beispielsweise über PCR-basierte oder auch synthetische Verfahren, ist dagegen einfach vorzunehmen.

Die Verwendung von PCR-Wildtyp-Fragmenten in Kombination mit einem oder mehreren randomisierten Oligonukleotiden - anstelle beispielsweise des ausschließlichen Einsatzes von Oligonukleotiden - erhöht die Flexibilität des Verfahrens. So können z.B. bei längeren Genen Restriktionsschnittstellen im Vektor gewählt werden und es müssen keine stillen Mutationen für die Linearisierung des Vektors erzeugt werden.

Sollen mehrere Mutationen vorgenommen werden, die in verschiedenen Bereichen des zu mutagenisierenden Gens liegen, können oft die Vektorprimer beibehalten werden. Auch das macht dieses Verfahren einfach und kostengünstig.

In dem erfindungsgemäßen Verfahren sind die randomisierten Oligonukleotide vorzugsweise einzelsträngig und enthalten die gewünschten Randomisierungen/Mutationen an einer oder mehreren Stellen. So wird vorteilhafterweise vermieden, mittels einer PCR den komplementären Strang synthetisieren zu müssen.

Es ist ein besonderer Vorteil des erfindungsgemäßen Verfahrens, dass die gewünschte DNA-Bank direkt in dem Ziel(Wirts)organismus und nicht vorab durch eine PCR mit den beschriebenen Nachteilen *in vitro* konstruiert wird. Darüber hinaus wird - sofern nur ein oder wenige randomisierte Oligonukleotide verwendet werden - die Gefahr einer Potenzierung von Fehlern, die bei der Synthese dieser Oligonukleotide geschehen können, begrenzt.

Weiterhin ist es vorteilhaft, dass für die PCR-basierte Amplifikation der Wildtyp-Fragmente keine degenerierten Primer eingesetzt werden. Dadurch kann die Entstehung von Mutanten, die dem Wildtyp ähnlich sind (Primer binden am besten an eine möglichst ähnliche Sequenz), vermieden werden. Ein ähnlicher Effekt, der bei der homologen Rekombination (beispielsweise) in Hefe auftreten kann, kann dadurch vermieden werden, dass die Überlappungsbereiche zwischen dem randomisierten Oligonukleotid mit den Fragmenten vorzugsweise keine Mutationen enthalten.

Vorteilhafterweise enthält die gewünschte DNA-Genbank keine originäre Wildtyp-Sequenz. Um eine solche "Verunreinigung" der gewünschten DNA-Genbank durch originäre Wildtyp-Sequenzen (Template-DNA) möglichst zu vermeiden oder zu minimieren, können die Wildtyp-Fragmente nach ihrer Generierung aus dem Gel eluiert und dann nur diese Fragmente zusammen mit dem (den) randomisierten Inserts in der Transformation eingesetzt werden.

In den Abb. wird exemplarisch die Position eines randomisierten Überbrückungsoligonukleotids dargestellt. Es können einzelne oder mehrere Positionen (Codons oder einzelne Basen) des Überbrückungsoligonukleotids randomisiert sein.

### 1. Prinzip des erfindungsgemäßen Verfahrens

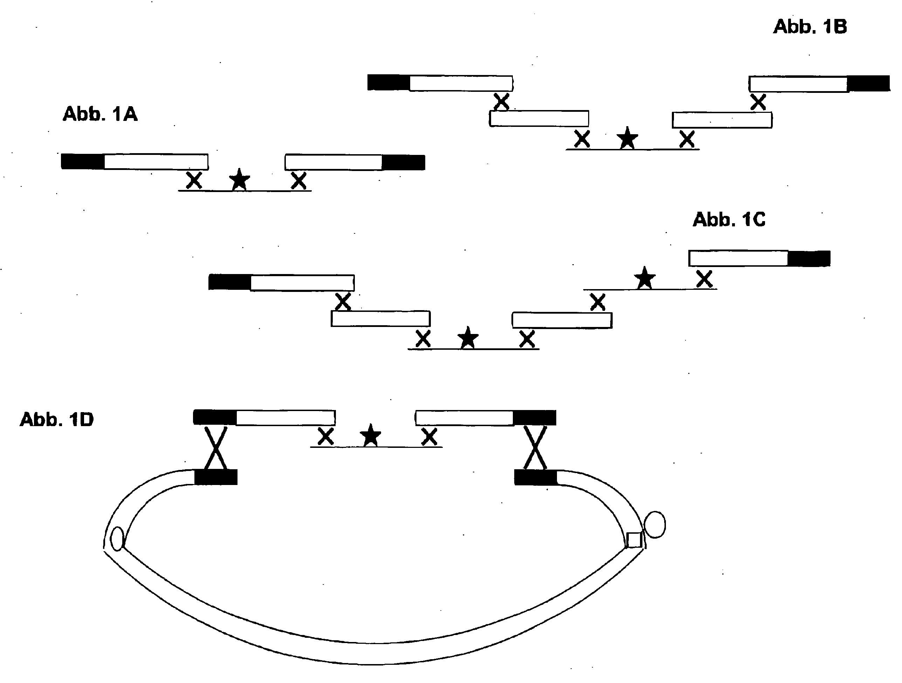

In Abb. 1 ist das Prinzip des erfindungsgemäßen Verfahrens dargestellt. Die Sterne symbolisieren (unterschiedliche) randomisierte Bereiche des Überbrückungsoligonukleotids, die grauen Balken stellen Vektorbereiche bzw. Homologien zu Vektorbereichen dar. Die hellen Balken geben Abschnitte des Gens wieder, das durch die Überbrückungsoligonukleotide mutagenisiert werden soll. Diese "hellen" Genabschnitte entsprechen jedoch den jeweiligen Wildtyp-Bereichen und sind daher selber nicht randomisiert. Die Kreuze symbolisieren die Überlappungsbereiche und kennzeichnen daher den Bereich der homologen Rekombination.

Die Abb. 1A und 1 B zeigen verschiedene Ausführungsformen der Erfindung zur Einbringung des (der) randomisierten Oligonukleotide. In den Abb. 1A und 1B ist das Verfahren mit nur einem Überbrückungsoligonukleotid dargestellt, das beispielsweise ein oder mehrere randomisierte Codons enthalten kann. Abb. 1 B zeigt exemplarisch die Rekombination von vier Wildtyp-Fragmenten und einem randomisierten Oligonukleotid. Dabei kann es sich z.B. um drei oder mehr Wildtyp-Fragmente handeln. Auch ist der Einsatz von nur einem Wildtypfragment, das in den folgenden Schritten mit einem oder mehreren randomisierten Überbrückungsoligonukleotiden zusammen verwendet wird, möglich.

Abb. 1C zeigt die Kombination zweier Überbrückungsoligonukleotide mit vier Wildtyp-Fragmenten. Diese Abb. soll exemplarisch verdeutlichen, dass verschiedene Kombinationen in Anzahl und Länge der beteiligten Fragmente/Oligonukleotide möglich sind.

Auch eine Kombination von zwei randomisierten Überbrückungsoligonukleotiden untereinander mit einer unterschiedlichen Anzahl von Wildtyp-Fragmenten ist denkbar.

Abb. 1 D zeigt die Integration der Situation in Abb. 1A durch homologe Rekombination in einen Vektor.

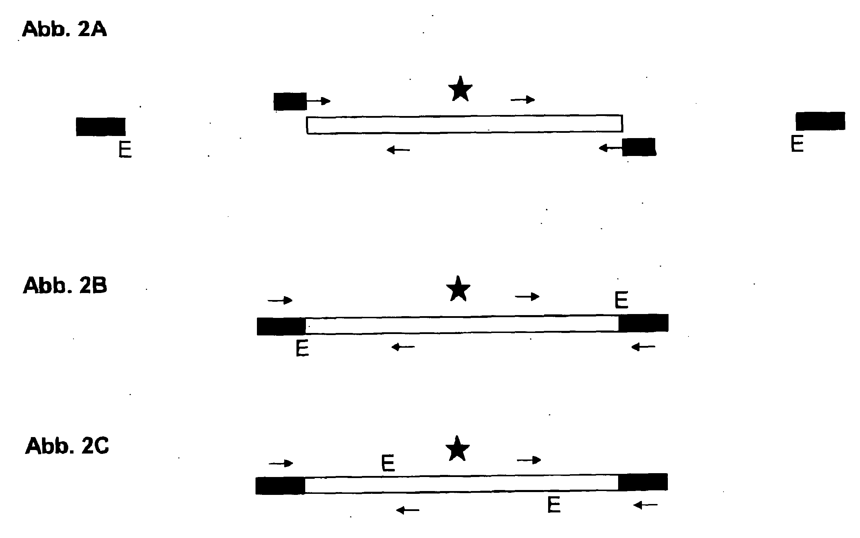

Abb. 2 zeigt, wie die Struktur des Integrationsvektors und der Wildtyp-Fragmente für die PCR zur Erzeugung der Wildtyp-Fragmente für die homologe Rekombination aussehen könnte. Die Steme symbolisieren die Position (eines) randomisierten Codons, die grauen Balken zeigen die Vektorbereiche bzw. die Homologien zu Vektorbereichen an, die hellen Balken kennzeichnen die Wildtyp-Bereiche des zu mutagenisierenden Gens und E steht für die Erkennungssequenz eines Restriktionsenzyms.

Abb. 2A verdeutlicht den Fall, dass das zu mutagenisierende Gen nicht in dem Vektor vorliegt, der in der Transformation eingesetzt wird. Hier können die äußeren Primer, die für die Synthese der PCR-Fragmente eingesetzt werden, einen Bereich besitzen, der homolog zum Vektor ist. Der Vektor kann mit dem Enzym E geschnitten werden.

Der Vorteil dieser Ausführungsform ist, dass keine Klonierung des zu mutagenisierenden Gens in den Vektor vorgenommen werden muss. Die verwendeten äußeren Primer sind vorteilhafterweise eher lang, um eine effektive Rekombination zu gewährleisten.

Abb. 2B zeigt die Situation, wenn das zu mutagenisierende Gen in dem Vektor vorliegt, der in der Transformation eingesetzt wird.

Vorteilhaft an dieser Situation ist, dass durch die Wahl der Primer der Homologiebereich zwischen dem Vektor mit Wildtyp-Fragmenten und dem mutagenisierten Genfragment beliebig groß gewählt werden kann; die verwendeten Primer müssen nicht, wie in Abb. 2A dargestellt, den Homologiebereich für die Rekombination *in vivo* enthalten.

In Abb. 2C ist dargestellt, wie diese Situation nach Einfügung von stillen Mutationen zur Erzeugung von Restriktionsschnittstellen (E) aussieht. Die Auswahl der stillen Mutationen kann mittels eines frei verfügbaren Computerprogramms (z.B. http://watcut.uwaterloo.ca/watcut/watcut/template.php) erfolgen. Diese Stellen können eingefügt werden, um die Auswirkung eines längeren Homologiebereichs zwischen Fragmenten und Vektor auf die Effizienz des erfindungsgemäßen Verfahrens festzustellen.

Vorteilhafterweise kann zur Vermeidung von Wildtyphintergrund ("Verunreinigung"; siehe oben) in der gewünschten DNA-Bank in die Restriktionsschnittstellen E oder in andere passende Restriktionsschnittstellen innerhalb des Gens oder des Vektors ein sogenanntes "stuffer-Fragment" (Sneeden und Loeb, 2003) einkloniert werden, das eine dem zu mutagenisierenden Gen völlig fremde Sequenz hat. Wird nun der Vektor, der das stuffer-Fragment enthält, mit den Klonierungsenzymen geschnitten, ist einerseits die Bande des vollständig verdauten Vektors gut von dem einmal geschnittenen Vektor auf dem Gel zu unterscheiden; andererseits entsteht bei der Transformation des Vektorfragmentes in den Wirtsorganismus kein durch Religationen verursachter Wildtyphintergrund, da der Vektor mit stuffer-fragment nicht funktionell ist.

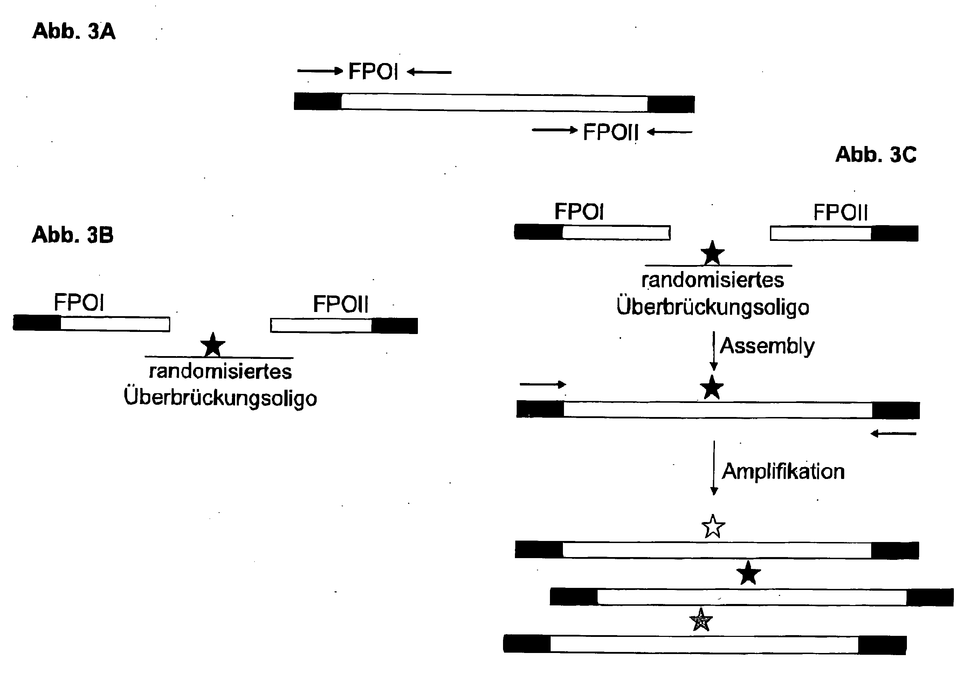

In der Abb. 3 ist das erfindungsgemäße Verfahren dargestellt (FPO steht für "Fragment plus Oligonukleotid"). Bei den dunkel dargestellten Bereichen handelt es sich um die Homologiebereiche zwischen dem Vektor und der Wildtyp-DNA oder dem randomisierten Überbrückungsoligonukleotid; die Sterne symbolisieren das randomisierte Codon. Bei der Generierung der Wildtyp-Fragmente kann eine Proofreading-Polymerase eingesetzt werden. Dadurch werden Fehler, die durch Einbau falscher Basen entstehen, minimiert.

Abb. 3A zeigt die Synthese zweier Wildtyp-Fragmente. Für dieses Verfahren können auch eins oder mehr als zwei Fragmente synthetisiert und für die folgenden Reaktionen eingesetzt werden. Ebenfalls ist es möglich, auch mehrere Überbrückungsoligos einzusetzen.

Eine weitere Möglichkeit ist die Synthese von nur einem Wildtypfragment, das in den folgenden Schritten mit einem oder mehreren randomisierten Überbrückungsoligonukleotiden zusammen verwendet wird.

Der nächste Schritt ist die Transformation der beiden Wildtyp-Fragmente mit dem Überbrückungsoligonukleotid, das an einer oder mehreren Stellen randomisierte Codons oder Basen tragen kann (Abb. 3B), in den Wirtsorganismus.

Das erfindungsgemäße Verfahren kann z.T. *in vitro* überprüft werden. Dazu kann ein Assembly mit dem Oligonukleotid in einer primerlosen PCR erfolgen, wie in Abb. 3C dargestellt. Die Produkte dieser Reaktion werden dann als Template für eine Amplifikation mit den beiden äußeren Primem eingesetzt; anschließend erfolgt dann die Transformation der Amplikons zusammen mit dem geschnittenen Vektor in die Hefezellen, in denen dann die *in vivo* Rekombination stattfinden kann.

In dem erfindungsgemäßen Verfahren muss das Überbrückungsoligonukleotid weder an eine Template-DNA binden, noch müssen sich die Mismatches im Homologiebereich befinden. Daher besteht viel Freiheit für die Position(en) der Randomisierung, weil sie die homologe Rekombination in dem Wirtsorganismus nicht beeinflussen.

Durch den Einsatz mehrerer Überbrückungsoligonukleotide (s. auch Abb. 1 C) können auch an weiter entfernten Positionen gleichzeitig mehrere Mutationen eingefügt werden.

Im Gegensatz zum reinen Gene Assembly, bei dem ein Gen aus einzelnen Oligonukleotiden aufgebaut wird, sind hier nur wenige Oligonukleotide nötig, was die Methode sowohl günstiger als auch weniger fehleranfällig macht, weil nicht die PCR-Reaktion, sondern die Qualität der Oligonukleotide oft den begrenzenden Faktor dieser Gene Assembly Methoden darstellt.

Zusätzlich ist in diesem Fall das Annealing zwischen den Fragmenten erleichtert, da es nur wenige rekombinogene Enden für eine in vivo-Rekombination gibt.

### 2. Vortests für das erfindungsgemäße Verfahren

### a) Erzeugen einer Genbank durch homologe Rekombination

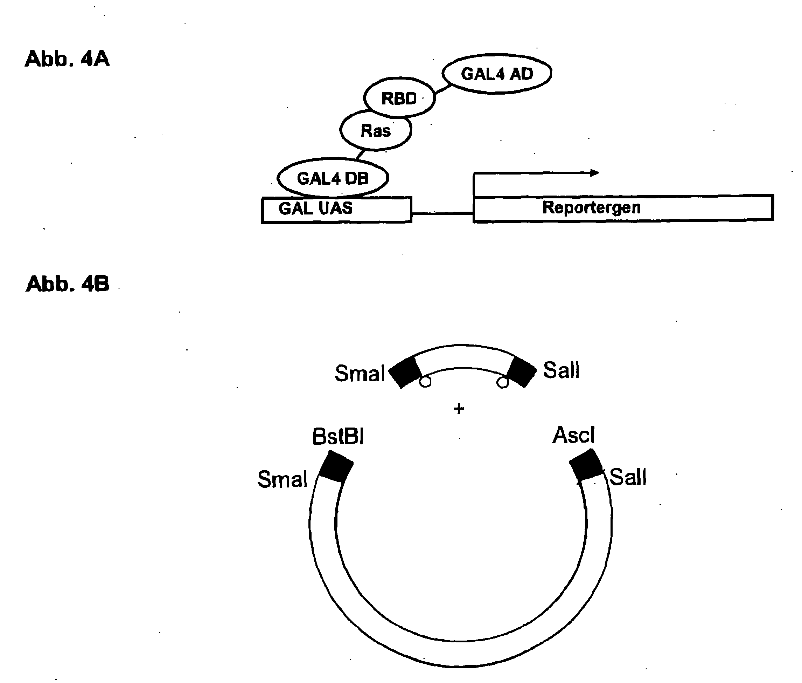

In diesem Test sollte zunächst nachgewiesen werden, dass das Prinzip der homologen Rekombination zur Erzeugung einer DNA-Bank einsetzbar ist. Dazu wurde ein Two-Hybrid System verwendet, das die Interaktion zwischen Ras und der Ras-bindenden Domäne (RafRBD) der Proteinkinase Raf (s. Abb. 4A) untersucht.

In den beiden im Folgenden beschriebenen Vortests macht man sich zunutze, dass das zu erwartende Ergebnis schon bekannt ist. Die homologe Rekombination *in vivo* wird also im Zusammenhang mit einer Bankengenerierung im Hinblick auf Funktionalität überprüft.

Das bekannte Two-Hybrid System wurde mit Hilfe von dem Fachmann geläufigen Verfahren durchgeführt.

### (i) Identifikation (Hitpicking") der bekannten RafRBD-Mutanten

Von der RafRBD sind verschiedene Mutanten bekannt (Block et al., 1996), die sich in ihren Bindungsaffinitäten unterscheiden, wobei sie sich wie folgt nach steigender Bindungsaffinität ordnen lassen:
RafRBD-R67A < T68A < V69A < WT (Wildtyp)

Zusätzlich ist noch eine Mutante A85K bekannt (Fridman et al., 2000), die in ihrer Bindungsaffinität über dem WT anzusiedeln ist. Werden diese Mutanten unter den bei Jaitner et al. (1997) beschriebenen Expressionsbedingungen in dem bekannten Two-Hybrid System untersucht, bestätigt sich diese Rangfolge, wobei die Abstufung zwischen WT und A85K nicht zuverlässig erfolgt.

Als Nachweis der Eignung der homologen Rekombination zur Generierung von DNA-Banken wurden stille Mutationen (BstBI/AscI) in das WT-RafRBD-Gen eingefügt und ein stuffer-Fragment einkloniert. Nachfolgend konnte der Vektor mit diesen Enzymen geschnitten und zur Transformation eingesetzt werden (s. Abb 4B).

Die beschriebenen mutierten RafRBD-Gene (R57A, T68A, V69A, A85K) sowie das Wildtyp-Gen wurden mit Smal/Sall vollständig aus ihren Vektoren geschnitten (s. Abb 4B) und aus dem Gel eluiert.

Vektor und Inserts wurden im molaren Verhältnis von 1:4 zur Hefetransformation eingesetzt; dabei wurde insgesamt 1 µg DNA transformiert. Ein molares Verhältnis von 1:6 ergab keine Verbesserung der Transformationsrate, die bei 2 bis 5 x 10³ pro µg DNA lag.

**Tab. 1: Ergebnis des Hitpickings der bekannten RafRBD-Mutanten. Als Fluorophor wurde RedStar eingesetzt.**

| | Ergebnis des quantitativen Screenings gepickte Mutante | | | |
|---|---|---|---|---|
| | A85 = Alanin = Wildtyp | | A85K = Lysin | |
| Beschreibung Ansatz | Anzahl | % | Anzahl | % |
| alle Mutanten-Fragmente gemischt plus geschnittener Vektor | 12 | 24,5 | 37 | 75,5 |
| zirkuläre Vektoren aller Mutanten gemischt | 50 | 76,9 | 15 | 23,1 |

Wie aus Tabelle 1 zu ersehen - und für das Two-Hybrid System zu erwarten war - sind nur der Wildtyp und die verbesserte Mutante A85K detektiert worden. Damit bestätigt sich die Einsetzbarkeit der homologen Rekombination für die Generierung von Banken *in vivo*.

### (ii) Identifikation von Mutanten ("Hitpicking") unter Einsatz eines Fragmentes mit randomisiertem Codon

Das randomisierte Codon wurde über eine Zufallsmutagenese nach der Methode von Zheng et al. (2004) an der Aminosäureposition 85 der RafRBD durchgeführt (siehe dazu im Einzelnen unter Ziffer 6). Im Wildtyp befindet sich hier ein Alanin. Aus der Literatur (Fridman et al., 2000) ist die deutlich verbesserte Mutante A85K (s.o.) sowie die Mutante A85R bekannt, die in ihrer Affinität für Ras zwischen WT und A85K liegt. Im Two-Hybrid System ist eine zuverlässige Unterscheidung dieser verbesserten Mutanten vom Wildtyp nicht möglich (eigene Daten, unveröffentl).

Um eine zuverlässige Diskriminierung zwischen diesen beiden Mutationen zu erreichen, wurde das sog. n-Hybrid System verwendet. Bei diesem System handelt es sich um eine Weiterentwicklung des bekannten Two-Hybrid Systems, der der Gedanke zugrundeliegt, das bekannte Hybrid-System so durchzuführen, daß die Bindungsreaktion zwischen dem ersten Liganden (erstes Hybrid- oder Fusionsprotein) und dem zweiten Liganden (zweites Hybrid- oder Fusionsprotein) durch die Wahl geeigneter Reaktionsbedingungen bewusst "verschlechtert" wird. Eine "Verschlechterung" ist immer dann gegeben, wenn die Lage des Gleichgewichts (Fließgleichgewichts) der Reaktion der Bildung eines Ligandenkomplexes zu Gunsten der Liganden (Ausgangsprodukte) verschoben wird. Die Bindungsreaktion zwischen den Liganden ist daher gehemmt bzw. verlangsamt. Der Ligandenkomplex ist gegenüber den Liganden durch einen funktionalen Transkriptionsfaktor definiert.

Wird einer der Liganden des Ausgangssystems jedoch z.B. durch einen Liganden mit einer deutlich höheren Affinität zu dem jeweils anderen Bindungspartner ersetzt, wird die "Verschlechterung" des Systems überwunden und es bilden sich im Vergleich zu der Ausgangslage entsprechend mehr Ligandenkomplexe. Die Verwendung eines höher affinen Liganden führt daher gegenüber der gestörten Ausgangslage zu einer verstärkten Expression des Reportergens, dessen Aktivität quantitativ nachweisbar ist. Dies ermöglicht insbesondere die Darstellung der relativen Affinität eines Interaktionspaares gegenüber einem Vergleichspaar.

Diese n-Hybrid System eignet sich vor allem als Screening-Verfahren zum Vergleich affiner und hochaffiner Liganden. Um diesen Vergleich zu ermöglichen, kann z.B. die Reportergen-Expression eines Wildtyp-Liganden als Referenzwert (100 %) angesetzt werden. Die Affinitäten von Mutanten dieses Wildtyps lassen sich davon ausgehend als relative Größen zu der Affinität des Wildtyps darstellen.

In der hier gewählten Ausführungsform des n-Hybrid Systems wird ein Kompetitor zur "Verschlechterung" der Bindungsreaktion eingesetzt. Der Kompetitor bindet an eines der Hybridproteine und hemmt oder verzögert damit im Sinne eines kompetitiven oder nicht-kompetitiven Hemmstoffes die Ausbildung des Ligandenkomplexes. Erfindungsgemäß ist es möglich, einen Kompetitor sowohl für das Beute- als auch für das Köderprotein einzusetzen. Sollte ein Kompetitor für das Beuteprotein gewählt werden - und somit die Ausbildung des Ligandenkomplexes dem Grunde nach verschlechtert werden - sollte aber die Affininität des Beuteproteins zu dem Köderprotein die Affinität des eingesetzten Kompetitors zu dem Köderprotein übersteigen, wird das Gleichgewicht der Ligandenbindungsreaktion wieder zu Gunsten der Ligandenkomplexbindung beeinflusst. Damit wird das hochaffine Beuteprotein durch die entsprechend hohe Reportergen-Expression quantitativ nachweisbar. Diese hochaffine Reaktion wäre nicht nachweisbar, wenn die Nachweisgrenze des Systems bereits überschritten wäre.

Für den hier geschilderten Vortest wird das Wildtyp-RafRBD ohne Fusionsprotein an die GAL4-Aktivierungsdomäne als Kompetitor eingesetzt. Dieses Protein bindet an Ras und bildet mit diesem einen inaktiven Komplex. Es konkurriert mit den Mutanten um die Bindung und gestattet so eine gesteigerte Diskriminierung zwischen verbesserten Mutanten von RafRBD und Wildtyp-RafRBD.

Prinzip und Durchführung des n-Hybrid Systems werden unter Ziffer 4 im Detail beschrieben.

Aus der Bank-DNA wurde mit Smal/Sall das Insert, das in diesem Fall ein randomisiertes Codon an Position 85 der RafRBD enthält, ausgeschnitten und nach einer Gelelution mit dem BstBI/AscI geschnittenen Vektor in Hefe transformiert (s. Abb. 4B).

Vektor und Insert wurden im molaren Verhältnis von 1:4 zur Hefetransformation eingesetzt; dabei wurden insgesamt 1 µg DNA transformiert.

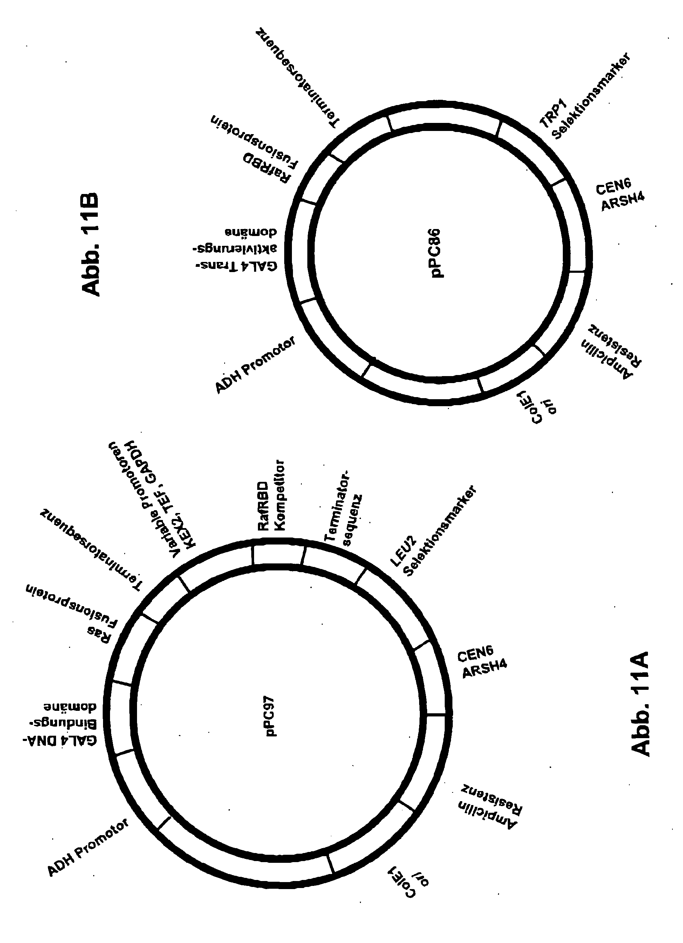

Bei allen Ansätzen wird entweder pPC97-ras (für das Two-Hybrid) oder pPC97-ras mit Kompetitor TEF-Promotor/RafRBD (für das n-Hybrid System, s. Abb. 11) als zweites Plasmid kotransformiert.

Unter Einsatz der homologen Rekombination *in vivo* konnte durch den Einsatz eines quantitativen Screenings mit Hitpicking der am stärksten fluoreszierenden Klone eine Diskriminierung zwischen dem Wildtyp und den verbesserten Mutanten erreicht werden (s. Tab. 1).

**Tab. 2: Ergebnis des Hitpickings. Gegenübergestellt sind die Ergebnisse des Hitpickings bei Einsatz des Two-Hybrid und des n-Hybrid Systems. Als Fluorophor wurde RedStar eingesetzt.**

| | Two-Hybrid | | n-Hybrid | |
|---|---|---|---|---|
| Hit | Anzahl | % | Anzahl | % |
| K (Lys) | 10 | 25 | 18 | 45 |
| R (Arg) | 13 | 32,5 | 19 | 47,5 |
| A (Ala) | 7 | 17,5 | 2 | 5 |
| P (Pro) | 5 | 12,5 | | |
| G (Gly) | 2 | 5 | | |
| S (Ser) | 2 | 5 | | |
| je 1 x | Q | 2,5 | T | 2,5 |

Aus Tab. 1 wird deutlich, dass die homologe Rekombination in beiden Systemen funktioniert hat und erwartungsgemäß (und vergleichbar mit dem Einsatz eines zirkulären Vektors, eigene Daten, unveröffentl.) im n-Hybrid die besseren Ergebnisse liefert: Bei Einsatz des n-Hybrid Systems zeigt sich eine viel geringere Variabilität im Vergleich zum Two-Hybrid System: die erwarteten Mutanten A85R und A85K wurden zu über 90 % gefunden.

Als Negativkontrolle wurde der Vektor alleine transformiert; hier waren wie erwartet keine Kolonien zu erkennen.

Die Sequenzen der Klone, die nach homologer Rekombination erhalten wurden, wurden genauer analysiert und zeigten alle eine präzise Rekombination. Dieses war zu erwarten, da nur die Klone, in denen die Rekombination basengenau erfolgt, in der Lage sind, auf dem Selektivmedium mit Aminotriazol als kompetitivem Hemmstoff für die His-Expression zu wachsen. Bei gleicher Protein-Protein-Interaktion korrespondiert der Reportergen-Readout mit dem hohen Selektionsdruck auf dem His3-Gen. Durch die Zugabe von 50 mM Aminotriazol werden nur Wirtszellen mit ausreichend hochaffinen Fusionsprotein selektiert. Die Affinität der Bindungspartner muss nämlich so groß sein, dass trotz des Wachstums auf einem kompetitiven His-Expressionshemmstoff noch eine ausreichende Menge His (bzw. das Reportergen) exprimiert wird.

In dieser besonders vorteilhaften Ausführungsform des n-Hybrid Systems findet also eine doppelte Selektion der Klone statt: Es wird auf das Vorhandensein des Vektors mit seinem Wachstumsmarker und parallel auf die erfolgreiche Rekombination eines der beiden Bindungspartner selektiert.
b) Einsatz von *in vitro* Assembly der Fragmente und des randomisierten Überbrückungsoligonukleotids vor der Transformation in Hefe

In diesem Vortest soll der amplification bias gezeigt werden. Der Vortest folgt dem in Abb. 3C dargestellten Prinzip.

Für das Assembly *in vitro* wurden jeweils 20 ng oder 40 ng der synthetisierten Fragmente FPOI und FPOII mit einem vierfachen molaren Überschuss des randomisierten Überbrückungsoligonukleotids in einer primerlosen PCR zusammengefügt. Der Ansatz betrug 50 µl. Das PCR-Programm lautete wie folgt:
94°C 2 min, 30 x (94°C 30 sec, 60°C 30 sec, 72°C 60 sec), 72°C 10 min

In einem zweiten Experiment wurden 40 ng synthetisierte Fragmente eingesetzt und das Assembly bei 64,8 und 65,8°C durchgeführt.

Zur Amplifizierung des Assembly-Produktes wurde 1 µl des Assembly-Ansatzes für eine PCR eingesetzt. Als Primer wurden FPO-out-for und FPO-out-rev verwendet. Das Programm war das Gleiche wie für die Assembly-Reaktion. Es wurden jeweils 5 PCR-Ansätze durchgeführt und nach der Amplifikation vereinigt, um das Auftreten eventueller prominenter PCR-Produkte (amplification bias) zu minimieren.

Die nach dem *in vitro* Assembly erhaltenen PCR-Fragmente wurden sequenziert. Die Abb. 5A gibt exemplarisch das Ergebnis wieder. Die Verteilung der Basen ist relativ gleichmäßig aber nicht ideal, was auf das Auftreten eines amplification bias trotz der Durchführung von Parallelreaktionen zurückzuführen ist.

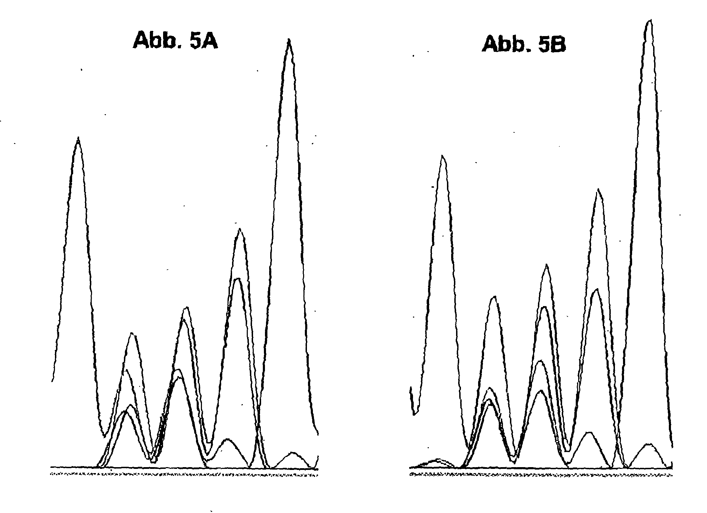

Nach dem Assembly *in vitro* wurde die DNA mit Xma/Sal restringiert, in den gleichermaßen geschnittenen Vektor ligiert und in Bakterien transformiert. Die DNA (= Bank-DNA) wurde aus den Bakterien mit dem Minipräp-Kit von Qiagen extrahiert und sequenziert. Die Abb. 5B gibt die Sequenzierungen wieder. Es ist deutlich zu erkennen, dass eine Base, in dem vorliegenden Fall ist es T, im Vergleich zu der Sequenz des PCR-Produktes (s. Abb. 5A) noch deutlicher überrepräsentiert ist. Dieses Phänomen, das wahrscheinlich auf den möglichen unterschiedlichen Wachstumseigenschaften der individuellen Klone beruht, wird auch von Schaerer-Brodbeck und Barberis (2004) beschrieben. Dieser Prozess kann den Verlust der Komplexität der Bank bedeuten.

Dieses Ergebnis zeigt noch einmal eindeutig, dass die homologe Rekombination *in vivo* dem Klonieren vorzuziehen ist und dass ein *in vitro* Assembly auch einen bias des PCR-Produktes zur Folge hat.

Die Bank-DNA (zirkuläres Plasmid) und die PCR-Produkte nach der Amplifikation der Assembly-Produkte wurden auch zur Hefetransformation eingesetzt (bei den Fragmenten wurde der geschnittene Vektor - verwendete Enzyme s. Tabelle 3 - zugegeben),

Bei allen Ansätzen wurde pPC97-ras mit Kompetitor TEF-Promotor/RafRBD (für das n-Hybrid System, s. Abb. 11) als zweites Plasmid kotransformiert.

**Tab. 3 Vergleich der Hitpicking-Ergebnisse. Assembly plus Vektor: Verwendung des PCR amplifizierten in vitro Assembly-Produktes für die in vivo Rekombination; zirkulärer Vektor: Verwendung der Banken DNA (zirkuläres Plasmid) für die Hefetransformation; DK = Doppelklon. Das verwendete Fluorophor ist in der ersten Zeile angegeben; die Restriktionsenzyme, mit denen der ggf. verwendete Vektor geschnitten wurde, sind in der zweiten Zeile vermerkt. Dabei erzeugt die Enzymkombination BstBI/AscI einen Überlappungsbereich von Fragment und Vektor von 153/130 bp; bei XmaI/SalI beträgt dieser 64/61 bp**

| | Fluorophor cobA zirkulärer Vektor 40 K (plus 7 DK) | | Fluorophor cobA BstBI/AscI Assembly plus Vektor 28 K (plus 2 DK) | | Fluor. RedStar zirkulärer Vektor 57 K (plus 3 DK) | | Fluor. RedStar XmaI/SalI Assembly plus Vektor 22 K (plus 1 DK) | |
|---|---|---|---|---|---|---|---|---|
| Hit | Anzahl | % | Anzahl | % | Anzahl | % | Anzahl | % |
| K (Lys) | 13 | 32,5 | 8 | 28,6 | 31 | 54,4 | 10 | 45,5 |
| R (Arg) | 24 | 60,0 | 17 | 60,7 | 23 | 40,4 | 12 | 54,5 |
| A (Ala) | | | | | | | | |
| P (Pro) | | | | | 1 | 1,8 | | |
| G (Gly) | 2 | 5,0 | 3 | 10,7 | 1 | 1,8 | | |
| S (Ser) | 1 | 2,5 | | | 1 | 1,8 | | |
| Rest | | | | | | | | |

Aus der Tabelle 3 geht hervor, dass der Einsatz von zirkulärem Vektor und Fragment plus Vektor (FPO) zu vergleichbaren Ergebnissen im Hitpicking führt; die homologe Rekombination funktioniert also genauso gut wie der Einsatz von zirkulärem Plasmid. Die verbesserten Mutanten werden, wie erwartet, bei Einsatz des n-Hybrid Systems (vgl. Tab. 2), zu rund 90 % wieder gefunden.

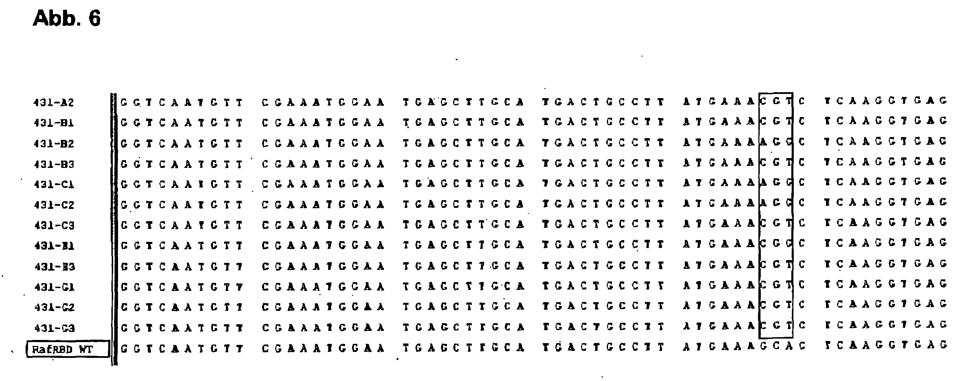

Eine Analyse der Sequenzen im Fall der Verwendung von RedStar und dem Einsatz von Fragment plus Vektor (Tab. 3, rechte Spalte) ist ein bias der Codons für Arginin (R) zu beobachten. In 8 von 12 Fällen (= 66,7 %; 3 x AGG, 1 x CGG) wurde CGT gefunden (s. Abb. 6; dargestellt ist die Sequenz eines Teils der RafRBD von 12 Klonen im Vergleich zur Wildtypsequenz (WT = Alanin = GCA), der Kasten kennzeichnet die randomisierte Position). Erwarten würde man jedes Codon zu ca. 33 %.

Bei allen Ansätzen werden Doppelklone detektiert. Bei diesen Klonen lässt die Sequenzanalyse erkennen, dass sich zwei verschiedene Codons an dieser Stelle befinden. Hier sind also zwei zirkuläre Bank-Plasmide aufgenommen bzw. zwei verschiedene Fragmente in zwei aufgenommene restringierte Vektoren eingebaut worden.

Bei der Mutagenese eines anderen Gens (Einsatz von *in vitro* Erzeugung der Bank mit PCR und Passage über *E. coli*, eigene Daten, unveröffentl.) wurde der amplification bias direkt nachgewiesen. Glycin war die verbesserte Mutante, die siebzehnmal gefunden wurde. In diesem Fall codierte nur GGG diese Aminosäure, obwohl auch GGT (ebenfalls Einsatz von NNG/T als degeneriertes Codon) Glycin codiert. Eine Analyse der gesamten Sequenz aller siebzehn Klone zeigte, dass an anderer Stelle der Sequenz ein Fehler (wobble) aufgetreten war, der in allen Klonen wieder zu finden war. Dieses ist nur möglich, wenn der Fehler in einem der ersten Zyklen der PCR-Reaktion aufgetreten ist und dieses Template dann im Laufe der folgenden Zyklen amplifiziert wurde.

Dieses Ergebnis macht noch einmal deutlich, welche Auswirkungen der amplification bias haben kann. Dieser kann dazu führen, dass in der Bank einige Mutanten nur zu einem sehr geringen Prozentsatz vorhanden sind und es somit nicht sicher ist, dass sich tatsächlich alle möglichen Mutanten in statistischen Anteilen in dem Zielorganismus befinden - die Menge der zu screenenden Kolonien wird erhöht.

Dabei ist dieser Effekt bei den nur durch ein Codon codierten Aminosäuren wie Methionin und Tryptophan besonders drastisch.

Sind Mutanten nur selten vorhanden, werden diese bei der Analyse einer bestimmten Prozentzahl an Hits nur selten gefunden. So können einzelne Mutanten, wenn sie dann gegenüber häufigen, ebenfalls verbesserten Mutanten nur wenige Male beobachtet werden, der Detektion entgehen.

Auch Fehler in der PCR können sich so fortpflanzen - diese sieht man in anderen Fällen nicht, wenn es sich bei dem aufgetretenen Fehler nicht um einen Wobble, wie in diesem Fall, sondern um einen Fehler mit Auswirkungen auf den Leserahmen oder um den Einbau einer anderen Aminosäure handelt. Da diese Kolonien dann nicht wachsen, könnte dieses Phänomen die Transformationseffizienz deutlich senken. Im Fall des Einbaus einer fremden Aminosäure könnte dies auch das Ergebnis des Screenings verfälschen, da die ungewollte Mutation den Effekt der beabsichtigten Mutation beeinflussen könnte und diese so der Detektion entgehen könnte.

### 3. Durchführung des erfindungsgemäßen Verfahrens unter Einsatz eines robotergestützten Hitpickings

Das erfindungsgemäße Verfahren wurde in einem Two-Hybrid und in einem n-Hybrid System durchgeführt (siehe dazu im Einzelnen auch Ziffer 4).

### (i) Konstruktion der Vektoren

Zur Konstruktion des Vektors wurden stille Mutationen zur Erzeugung von BstBI und AscI-site in das RafRBD-Gen, das in dem Vektor pPC86-RafRBD mit KanamycinResistenz vorliegt, eingefügt. Hierzu wurden folgende Primer in einer QuikChange-Multi Reaktion eingesetzt:
QC-RafRBD-BstBI
AAGAACAGTGGTCAATGTTCGAAATGGAATGAGCTTG
QC-RafRBD-Ascl
ACGAACACAAAGGTAAAAAGGCGCGCCTAGATTGGAATACTGATGCTG
Die im Vergleich zur Wildtyp-Sequenz veränderten Basen sind in Fettdruck dargestellt.

Nach Verifizierung durch Restriktion und Sequenzierung wurde in diese Restriktionssites ein 2,6 kb großes stuffer-Fragment einkloniert, das mit entsprechenden Primern mit einem anderen Vektor als Template amplifiziert wurde.

Dieser Vektor mit stuffer-Fragment wurde dann mit BstBI/AscI oder Smal(oder Xmal)/Sall geschnitten und für die homologe Rekombination zusammen mit den Fragmenten/dem randomisierten Überbrückungsoligonukleotid in Hefe transformiert.

### (ii) Erzeugung der Wildtyp-Fragmente

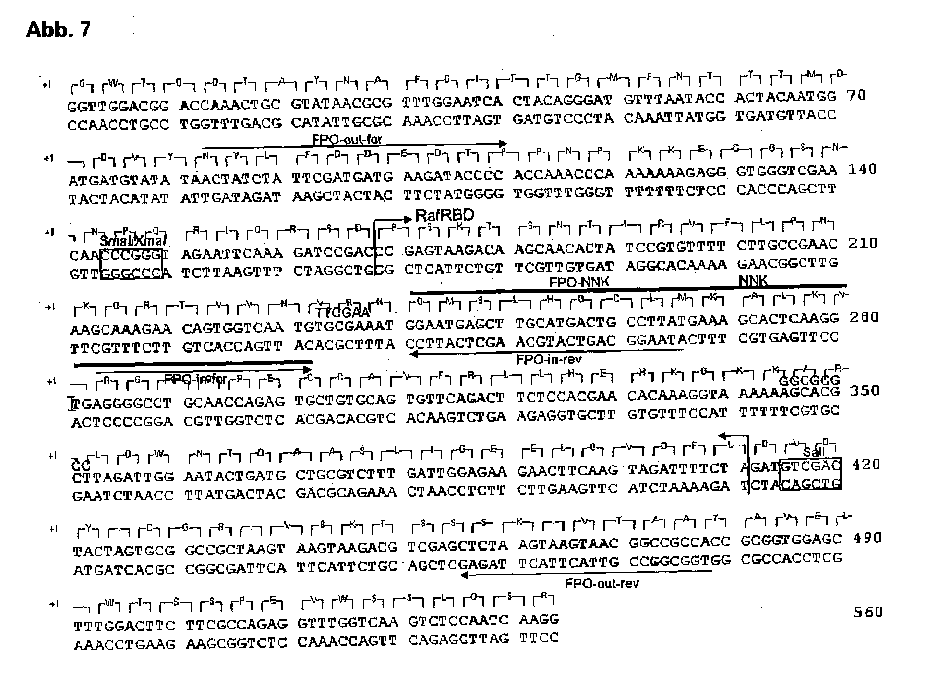

Abb. 7 zeigt das RafRBD-Gen mit der Lage der verwendeten Primer und des randomisierten Überbrückungsoligonukleotids. Die reverse Primer liegen unterhalb der Sequenz; die forward-Primer oberhalb der Sequenz. Die Nummerierung bezieht sich auf die Nukleotidsequenz und die durch stille Mutationen erhaltenen Sequenzen der Restriktionsschnittstellen (s.o.) sowie die anderen verwendeten Schnittstellen sind über der Sequenz dargestellt.

Die Primersequenzen lauten wie folgt:

| | |
|---|---|
| FPO-out-for | ACTATCTATTCGATGATGAAGATACCCC |
| FPO-out-rev | TGGCGGCCGTTACTTACTTAGAG |
| FPO-in-rev | ATAAGGCAGTCATGCAAGCTCATTCC |
| FPO-in-for | AGGGGCCTGCAACCAGAGTG |

FPO-NNK
GGAATGAGCTTGCATGACTGCCTTATGAAANNKCTCAAGGTGAGGGCCTGCA
ACCAGAGTG

FPO-NNK 3
AGAACAGTGGTCAATGTTCGAAATGGAATGAGCTTGCATGACTGCCTTATGAAA
NNKCTCAAGGTGAGGGGCCTGCAACCAGAGTGCTGTGCAGTGTTCAGACTTCT
CCACGAACA

Für die PCR wurden in einem 100 µl PCR-Ansatz bei der Erzeugung der beiden Fragmente FPOI und FPOII (s. Abb. 4) von den Primem FPO-out-for und FPO-in-rev (FPOI) bzw. FPO-in-for und FPO-out-rev (FPOII) je 40 pmol eingesetzt. Als Template wurden 30 ng des Vektors mit den stillen Mutationen im RafRBD-Gen verwendet. Die Konzentration der dNTPs betrug 200 µM und die Reaktion wurde in 1 x Pfu-Puffer mit 2,4 Units des Enzyms durchgeführt. Das PCR-Programm lautete wie folgt: 94°C 2 min, 30 x (94°C 30 sec, 60°C 40 sec, 72°C 40 sec), 72°C 5 min

Für das Assembly *in vivo* wurden insgesamt 500 ng DNA pro Transformationsansatz in die Hefe eingebracht und Vektor (BstBI/AscI oder SmaI/SalI geschnitten) und Fragmente/Oligo wurden im molaren Verhältnis von 1:4 eingesetzt. Dabei wurden die Fragmente FPOI, FPOII und das randomisierte Überbrückungsoligo transformiert.

Bei allen Ansätzen wird entweder pPC97-ras (für das Two-Hybrid) oder pPC97-ras mit Kompetitor TEF-Promotor/RafRBD (s. Abb. 11) als zweites Plasmid kotransformiert.

### (iii) robotergestützter Prozess

Der robotergestützte Prozess mit Scannen der Platten, Auswertung, Hitpicking, DNA-Isolation aus den Hefen und Transformation der Plasmide in die Bakterien wird im Detail unter Ziffer 5 dargestellt.

### (iv) Sequenzierung und Auswertung der Hits im Two-Hybrid System

**Tab. 4 Hltplcking-Ergebnisse Als Fluorophor wurde cobA verwendet. Die Restriktionsenzyme, mit denen der verwendete Vektor geschnitten wurde, sind vermerkt. Dabei erzeugt die Enzymkombination BstBI/AscI einen Überlappungsbereich von Fragment und Vektor von 153/130 bp; bei Xmal/Sall beträgt dieser 64/61 bp**

| | FPO I, FPO II, FPO-NNK 3 Vektor Xmal/Sall | | FPO I, FPO II, FPO-NNK Vektor BstBI/AscI | |
|---|---|---|---|---|
| Hit | Anzahl | % | Anzahl | % |
| K | 3 | 10,0 | | |
| R | 4 | 13,3 | 4 | 23,5 |
| A | 4 | 13,3 | 2 | 11,1 |
| P | 3 | 10 | 2 | 11,8 |
| G | 3 | 10 | 2 | 11,8 |
| S | 3 | 10 | 3 | 17,6 |
| Rest | 2xN,C,V 3xH,1xT 10 | 33,3 | H, Q, C, N | je 5,9 |

In beiden Versuchen werden die verbesserten Mutanten zu einem signifikanten Prozentsatz detektiert; allerdings treten neben den Mutanten, die im n-Hybrid System ebenfalls gefunden werden (s. Tab. 3), noch zusätzliche Aminosäuren auf. Dieses ist auf die geringere Selektivität des Two-Hybrids gegenüber dem n-Hybrid System zurück zu führen (s.o. und eigene Daten, unveröffentl.).

### (v) Sequenzierung und Auswertung der Hits im n-Hybrid System

**Tab. 5 Hitpicking-Ergebnisse (verbesserte Mutanten) Als Fluorophor wurde cobA verwendet. Die Restriktionsenzyme, mit denen der verwendete Vektor geschnitten wurde, sind vermerkt. Dabei erzeugt die Enzymkombination BstBI/AscI einen Überlappungsbereich von Fragment und Vektor von 153/130 bp**

| | FPO I, FPO II, FPO-NNK Vektor BstBI/AscI | |
|---|---|---|
| Hit | Anzahl | % |
| K | 7 | 87,5 |
| R | 1 | 12,5 |

Die Selektivität des n-Hybrid Systems ist gegenüber der des Two-Hybrid Systems deutlich erhöht (s.Tab. 3 und eigene Daten, unveröffentl.); bei den verbesserten Mutanten wird im Wesentlichen die Mutante mit der höchsten Affinität identifiziert. Ziel der Generierung einer mutagenisierten Bank ist die Detektion von verbesserten Mutanten. Dementsprechend wurde das erfindungsgemäße Verfahren auch im n-Hybrid System angewendet.

Ein gap repair kann stattfinden. Dieses Ereignis tritt ein, da das Wildtypgen als Kompetitor auf dem zweiten, kotransfomierten Plasmid vorhanden ist. Zur Vermeidung dieses gap repairs wurde die Synthese des Gens mit möglichst viel Divergenz (z.B. geänderte Codon Usage) zu der Originalsequenz durchgeführt. Bei Einsatz dieses Gens sollte keine Rekombination mehr möglich sein, was zu einem reduzierten Hintergrund an Wildtypsequenz führt. Eine weitere Möglichkeit zur Unterdrückung dieses Vorgangs ist der Einsatz von synthetischen Wildtypfragmenten, die eine Variabilität zu dem Wildtyp-Kompetitor aufweisen.

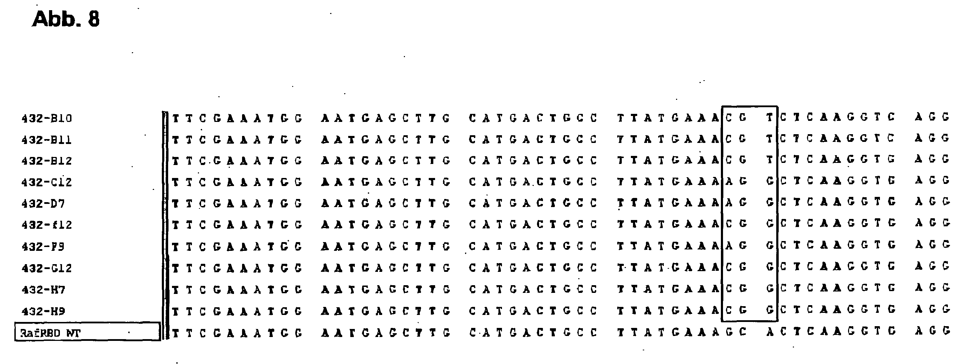

Analysiert man die Codonsequenzen für Arginin bei einem Parallelversuch, bei dem diese Aminosäure zehnmal detektiert wurde, so findet man im Gegensatz zu dem unter 2b beschriebenen bias der Codons für diese Aminosäure eine völlige Gleichverteilung der auftretenden Codons. AGG und CGT treten je dreimal auf, während CGG viermal vorhanden ist (s. Abb. 8; dargestellt ist die Sequenz eines Teils der RafRBD von 10 Klonen im Vergleich zur Wildtypsequenz (WT = Alanin = GCA), der Kasten kennzeichnet die randomisierte Position). Dieses Ergebnis zeigt, dass das erfindungsgemäße Verfahren geeignet Ist, eine Bank zu generieren, in der jedes Codon mit gleicher Häufigkeit zu finden ist.

In keinem der Ansätze, die unter Einsatz des erfindungsgemäßen Verfahrens durchgeführt wurden, wurden Doppelklone (zwei verschiedene Codons an der mutagenisierten Stelle, s. o.) detektiert. Dieses stellt eine Verbesserung gegenüber den Ansätzen mit zirkulärem Vektor oder mit dem durch *in vivo* assembly erzeugten Fragment dar, bei denen immer Doppelklone gefunden wurden (s. Tab. 3).

### 4. Durchführung des n-Hybrid Systems

### 1. Prinzip des n-Hybrid Verfahrens mit Kompetitor

Zusätzlich zu den interagierenden Hybridproteinen (Fusionsproteinen) des bekannten Two-Hybrid Systems wird in *S. cerevisiae* vorteilhafterweise eine dritte Komponente exprimiert. Dabei handelt es sich im Falle der Mutagenese des Köderproteins vorzugsweise um das freie Wildtyp-Köderprotein. Im Falle der Mutagenese des Beuteproteins zur Identifikation hochaffiner Beuteproteine wird vorzugsweise das Wildtyp Beuteprotein als Kompetitor exprimiert.

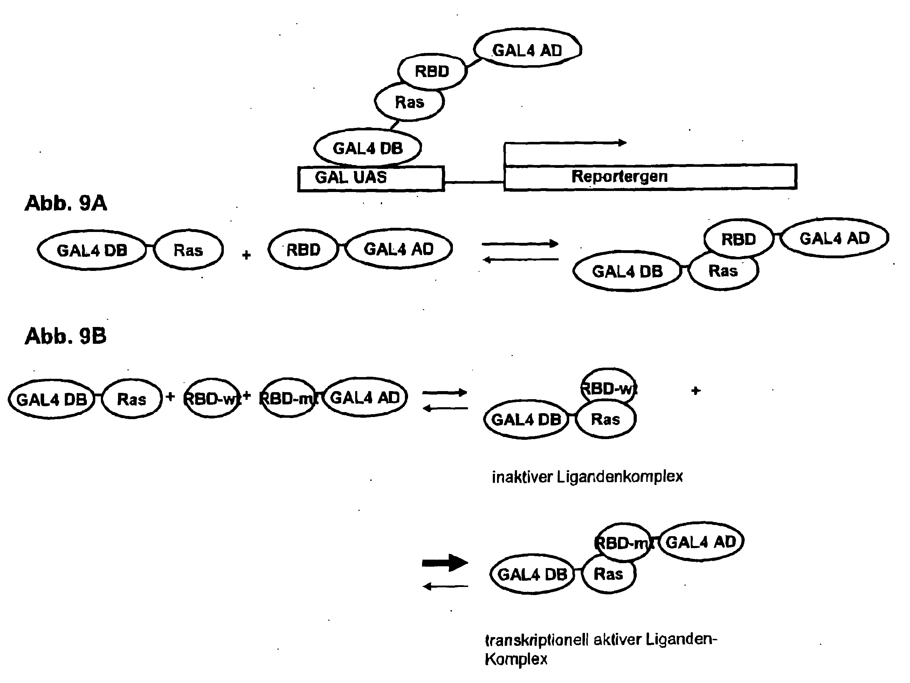

Das Grundprinzip des n-Hybrid Verrfahrens in dieser Ausführungsform ist in Abb. 9B für die Identifikation affiner RafRBD-Beuteproteine unter Expression des Wildtyp-Beuteproteins als Kompetitor dargestellt. Bei der Erhöhung der Affinität des mutierten RafRBD-Fusionsproteins (RBD-mt) wird die Bildung des transkriptionell funktionalen Ligandenkomplexes gegenüber dem nicht-funktionalen (inaktiven) Komplex aus dem Kompetitor RafRBD-wt (RBD-wt) mit Ras bevorzugt. [Abkürzungen: RBD-mt: mutiertes RafRBD-Fusionsprotein; RafRBD-wt: Wildtyp-RafRBD; RBD: Ras-bindende Domäne; DB: DNA-Bindungsdomäne; AD: Transaktivierungsdomäne; UAS: Upstream-Aktivatorsequenz]

Die Abb. 9A zeigt das bekannte Two-Hybrid System. In dem bekannten System wird nur die Wildtyp-Variante des Beuteproteins in dem Fusionsprotein eingesetzt. [Abkürzungen: RBD; Ras-bindende Domäne; DB: DNA-Bindungsdomäne; AD: Transaktivierungsdomäne; UAS; Upstream-Aktivatorsequenz]

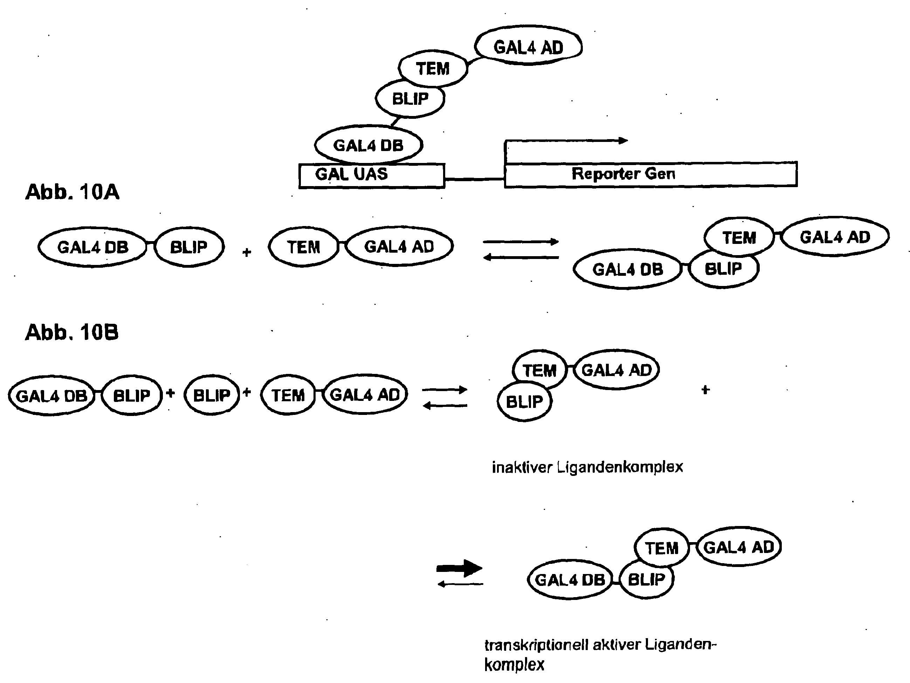

Das Verfahren nach Abb. 10B dient der Untersuchung der Interaktion zwischen den Proteinen BLIP als Köderprotein und TEM als Beuteprotein. Das Köderprotein wurde zuvor mutagenisiert (BLIP-mt) und als Fusionsprotein eingesetzt. In diesem System wird zusätzlich Wildtyp-BLIP (BLIP-wt) exprimiert und dient als Kompetitor. Bei Erhöhung der Affinität des mutierten BLIP-Fusionsproteins wird die Bildung des transkriptionell funktionalen (aktiven) Ligandenkomplex gegenüber dem inaktiven Ligandenkomplex aus BLIP-wt und dem Fusionsprotein mit TEM bevorzugt. [Abkürzungen: DB: DNA-Bindungsdomäne; AD: Transaktivierungsdomäne; BLIP-wt: Wildtyp des Köderproteins; BLIP-mt: mutiertes Köderprotein; UAS: Upstream Aktivatorsequenz]. Die Raf-RBD-Fusions-Beuteproteine wurden zuvor über eine dem Fachmann bekannte Mutagenese erzeugt. Grundsätzlich stehen für die Mutagenese alle üblichen Verfahren zur Verfügung.

Als Reportergene für dieses quantitative Screening können beispielsweise Met1, CysG oder CobA eingesetzt werden (Roessner, C.A. 2002), die jeweils fluoreszente Derivate von Uroporphyrinogenlll liefern. Es ist auch der Einsatz von fluoreszenten Proteinen als Repörtergen möglich, wie z.B. der Einsatz von Phycocyanin (Amtz et al., 2004) oder RedStar (Knop et al., 2002).

Zur Demonstration des Verfahrensprinzips für die Kombination der Mutagenese des Beuteproteins unter Expression des Wildtyp-Beuteproteins als Kompetitor wurde die RafRBD Mutante RafRBD-A85K eingesetzt, deren Bindungsaffinität bereits biochemisch charakterisiert ist. Die Dissoziationskonstante der RafRBD-A85K Mutante beträgt nach eigenen unveröffentlichten mikrokalorimetrischen Messungen in PBS-Puffer 72 nM im Vergleich zur Dissoziationskonstanten des entsprechenden RafRBD-wt Proteins von 253 nM.

Zur Untersuchung der Ras/Raf-Interaktionen im n-Hybrid Verfahren kann zur Bereitstellung des Köder-Fusionsproteines Ras-Gal4 ein aus dem Plasmid pPC97 abgeleitetes Plasmid verwendet werden. Auf diesem Plasmid wird auch der Kompetitor codiert. Einen Überblick über ein mögliches Plasmid gibt Abb. 11 A. Die Abb. 11 B zeigt ein Plasmid auf Basis des pPC86, der das Beute-Fusionsprotein codiert.

### 2. Konstruktion von Hefestämmen für das n-Hybrid Verfahren

### a) Reportergene für das quantitative Screening

Für das n-Hybrid Verfahren wird ein Reportergen benötigt, das unter der Kontrolle eines regulierbaren Promotors steht und dessen Aktivität unmittelbar, qualitativ und quantitativ in der intakten Hefekolonie gemessen werden kann. Reportergene, die eine Fluoreszenz als Readout bewirken, erfüllen diese Ansprüche.

### (i) Wahl des Promotors für das Reportergen

In der Hefe Y190 wird die Expression des Reportergens β-Galaktosidase durch die Stärke der Wechselwirkung von GAL4 Binde- und Aktivierungsdomäne reguliert, deren Gene durch zwei verschiedene Vektoren (pPC86 und pPC97, Chevray und Nathans, 1992) in die Hefezelle eingebracht werden.

Der Genotyp des Hefestamms Y190 ist als: "Mat a, leu2-3, 112, ura3-52, trp1-901, his3-Δ200, ade2-101, gal4Δgal80Δ, URA3::GAL-lacZ, LYS2::GAL-HIS3, cyh'" bekannt. Dabei bedeutet URA3::GAL-lacZ, dass der fragliche Promotor (GAL; divergenter GAL1/GAL10-Promotor) in das URA3-Gen integriert worden ist.

Für das Ziel, ein Reportergen hinter diesen Promotor zu klonieren und in das Genom der Hefe zu integrieren, ist eine genaue Kenntnis der Situation an dieser Stelle im Genom unabdingbar (vgl. dazu Yocum et al. (1984) zur Integration des Ylp Plasmides pRY171 in das Genom von Y152 (entstanden aus YJ0-Z, Leuther und Johnston, 1992), der der Vorläuferstamm von Y153 ist, aus dem wiederum Y190 entstanden ist).

Dann wurde die Entstehung des Plasmides pRY171, das den GAL-Promotor mit dem lacZ-Gen, beides hinter dem URA3-Gen, trägt, entschlüsselt, um Sequenzdaten zu erhalten: Yocum et al. (1984) haben dieses Plasmid aus dem Plasmid pLRIΔ3 durch die Entfernung der 2 µm Replikationsursprung-Sequenzen generiert. pLRIΔ3 entspricht dem Plasmid pRY131 bis auf einen Xhol Linker in der Mitte des divergenten Promotors. pRY131 wurde von West et al. (1984) generiert aus pLG 669 (Guarente und Ptashne, 1981) und pRY116. pLG 669 wiederum ist aus YEp24 entstanden, einem Plasmid mit pBR322-Rückgrat (Botstein et al., 1979).

Aus diesen Daten wurde eine Sequenz generiert, nach der Primer (365-for 394 for und 1563-rev sowie 1674-rev) synthetisiert wurden. Mit diesen wurde in einer PCR mit genomischer DNA aus Y190 dieses Stück DNA amplifiziert und sequenziert. Hierdurch wurde die tatsächliche Sequenz des divergenten GAL10/GAL1-Promoters, hinter den das Reportergen zu klonieren war.

Mit diesen Primern wurden durch PCR die entsprechenden Stücke des Promotors amplifiziert und dann als Fusionsprodukt mit einem Fluorophor kloniert. Zusätzlich wurde noch der Promotor 365-1451 ausgewählt, der keinen IacI/5'IacZ-Anteil mehr besitzt. Dies geschah aus der Überlegung heraus, dass zusätzliche Genanteile die Expression des ausgewählten Fluorophors behindern könnten. Ein weiterer Promotor, bei dem auch der GAL1-Anteil auf Null reduziert war (365-1366), wurde ebenfalls getestet. Bei dem Fluorophor handelte es sich um RedStar (Knop et al. 2002: s. Abschnitt (iii)).

Als bester Promotor stellte sich das Konstrukt 365-1451 (kein IacI/5'-IacZ mehr vorhanden) heraus; dieses wurde für alle folgenden Integrationen von Reportergenen in das Genom von *S. cerevisiae* eingesetzt.

Verwendete Primer:

| | |
|---|---|
| 365-for | ACGGGTACCGCAAAGGGAAGGGATGCTAAGG (Kpnl) |
| 394-for | ATCGGTACCTGAACGTTACAGAAAAGCAGG (Kpnl) |
| 1563-rev | ACTACTAGTGCCTCTTCGCTATTACGCCAGC (Spel) |
| 1674-rev | AGAACTAGTGGAAGATCGCACTCCAGC (Spel) |
| 1451-rev | ACAACTAGTAACTTTTCGGCCAATGGTCTTG (Spel) |
| 1366-rev | ACTACTAGTCCTATAGTTTTTTCTCCTTGACGTTAAA (Spel) |

### (ii) FOA-Behandlung von S. cerevisiae Y190

Für das n-Hybrid Verfahren werden Reportergene benötigt, die in das Genom der Hefe integriert sind. Hierzu muss ein Selektionsmarker zur Verfügung stehen, so dass nur Transformanten, die tatsächlich das gewünschte Gen am richtigen Locus in das Genom integriert haben, wachsen können.

Der Hefestamm Y190 braucht also zusätzlich zu den Auxotrophie-Markem Leucin und Tryptophan, die durch das Two-Hybrid System belegt sind, noch einen zusätzlichen Marker. Hierfür bietet sich Uracil (URA3-Gen) an, da bei diesem Gen die Möglichkeit besteht, den Stamm für diese Substanz auxotroph zu machen.

Bei der Herstellung URA3 negativer Klone wird die natürliche Mutationsfrequenz der Hefe von etwa 10⁻⁴ zunutze gemacht. Um bei den Hefen auf die Mutationsereignisse selektionieren zu können, wird ein Medium verwendet, das FOA (5-fluoro-orotic acid) enthält (Treco DA, 1989). Hefezellen, die kein Uracil mehr produzieren, also den gewünschten Phänotyp aufweisen, überleben, während die Zellen ohne Mutation im URA3-Gen absterben (Boeke et al., 1984).

### (iii) RedStar

Als Marker wurde RedStar (RFP) als ein für die Nutzung in *Saccharomyces cerevisiae* optimiertes Fluorophor (Knop et al., 2002) eingesetzt.

Die Amplifikation von RedStar erfolgte mit folgenden Primem:
RedStar-for ACTACTAGTTATGAGTAGATCTTCTAAGAACGTC (SpeI)
RedStar-rev TATTCCGCGGTTACAAGAACAAGTGGTGTCTAC (Sacll)

Der jeweilige Promotor und das RedStar-Gen wurden in einer Drei-Fragmente-Ligation (25 fmol Vektor, 125 fmol Inserts) in pRS306 kloniert. Hierbei handelt es sich um einen Integrationsvektor. Auf die Integration von RedStar (oder aller anderen Reportergene unter der Kontrolle des GAL-Promoters) ins Genom der Uracil-auxotrophen Hefe Y190D (s. (ii)) kann mittels des Uracil-Markers von pRS306 selektiert werden.

### (iv) cob A

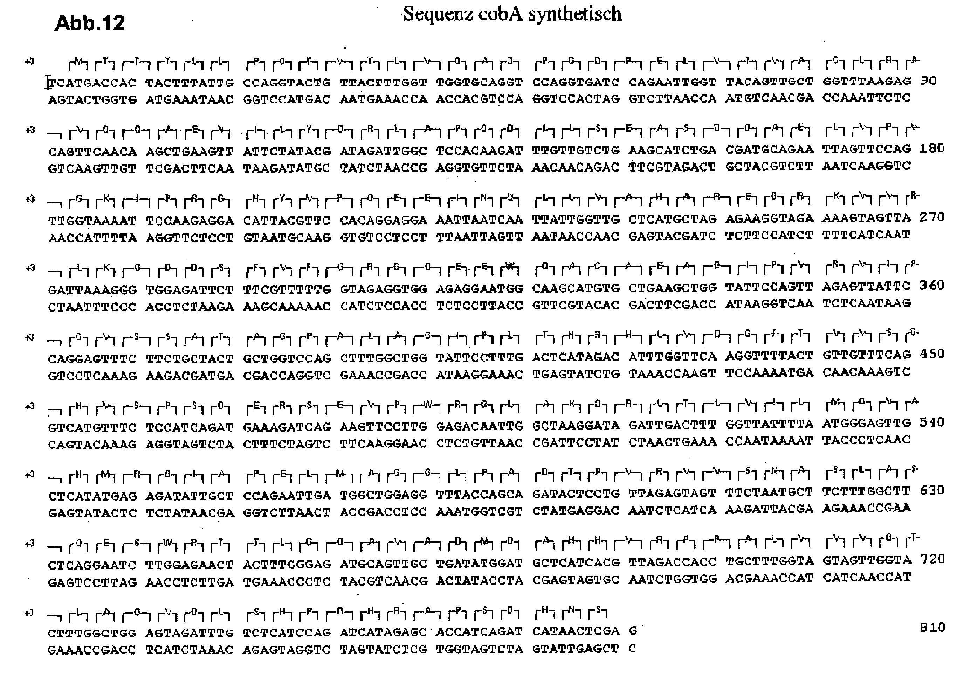

cob A codiert für Uroporphyrinogen III Methyltransferase aus *Propionibacterium freudenreichii*. Überexpression dieses Gens führt zu einer Fluoreszenz im Bereich von 605 nm, die auf die Akkumulation des fluoreszenten Produktes Trimethylpyrrocorphin beruht (Wildt und Deuschle, 1999). Eine Analyse des Codon Usage ergab einen hohen Prozentsatz an kritischen Codons bei der Expression des bakteriellen Gens in Hefe. Daraufhin wurde die Sequenz auf die Häufigkeit der Codonnutzung (Codon Usage) von *S. cerevisiae* optimiert und synthetisiert (Sequenz s. Abb. 12). Auch hier wurde das Gen nach dem Anfügen eines His-Tags und einer Terminationssequenz (s.o.) zusammen mit dem Promotor der Wahl (s.o.) über SacI/NotI in pRS306 kloniert und in das Genom der Hefe integriert.

### (v) Met 1

MET1 ist das entsprechende Protein aus *Saccharomyces*. Das 1,8 kb große Gen (Sequenz s. Abb 10) wurde aus dem Hefegenom mittels PCR amplifiziert.
Eingesetzte Primer:
Met1-for: AATTATCCATGGTACGAGACTTAGTGACATTG (Ncol)
Met1-rev: AATTAACTCGAGTTGTATAACTTAAATAGACTATCTACATCAACC (Xhol) Das Fragment wurde über Ncol/Xhol (Ncol enthält das Startcodon) in einen Vektor kloniert, der das Anfügen eines His-Tags und einer Terminationssequenz für Hefegene erlaubt (Amtz et al., 2004). Nach der Klonierung des Reportergens (Ncol/Notl) mit dem Promotor der Wahl (Sacl/Ncel) über Sacl/Notl in den Vektor pRS306 erfolgte die Integration des Reportergens in das Genom der Hefe.

### b) Durchführung des quantitativen Screenings im n-Hybrid Verfahren

Zu der Durchführung des quantitativen Screenings gehören die Herstellung des Mediums, die Transformation der Hefen und das Scannen der Platten. Dabei müssen alle Parameter standardisiert und optimiert sein, damit die Fluoreszenzergebnisse reproduzierbar sind.

### (i) Herstellung des Mediums

Für die Kultivierung und das Scannen der Hefen auf Fluoreszenz durch den LSA-Scanner werden folgende Medien benötigt:
YPAD-Medium (Vollmedium für Hefen)
5.0g Yeast extract (Difco)
10.0 g Peptone (Difco)
50 mg Adenine hemisulphate
ddH₂O ad 460 ml
pH vor dem Autoklavieren auf 5.8 einstellen; nach dem Autoklavieren hat das Medium einen pH-Wert von 5.6;
Für Agar-Platten: Zusatz 10 g Yeast-agar (Difco) nach der pH-Einstellung
Autoklavieren 15 Minuten bei 121°C;
Nach dem Autoklavieren werden 40 ml 25%ige Glucose (wird getrennt vom Medium autoklaviert) hinzugefügt.

Synthetisches Komplettmedium (ohne Leu, Trp, His)
3.35 g Yeast Nitrogen Base (w/o amino acids)
1 g Synthetic Complete Drop Out Mix (Aminosäuremix ohne Leu, Trp, His) ddH₂O ad 460 ml;
pH vor dem Autoklavieren auf 5.8 einstellen; nach dem Autoklavieren hat das Medium einen pH-Wert von 5.6;
Für Agar-Platten: Zusatz 10 g Yeast-agar (Difco) nach der pH-Einstellung
Autoklavieren 15 Minuten bei 121°C;

Nach dem Autoklavieren werden 40 ml 25%ige Glucose (wird getrennt vom Medium autoklaviert) und 10 ml 2.5 M 3-Amino-1,2,4-Triazol (sterilfiltriert) hinzugefügt.
Endkonzentration Glucose im Medium: 2 %
25%ige Glucose
100 g Glucose (Sigma)
400 ml ddH₂O
Autoklavieren 15 Minuten bei 121 °C
2.5 M 3-Amino-1,2,4-Triazol
1.051 g 3-Amino-1,2,4-Triazol
5 ml ddH₂O
Mit einem Sterilfilter (Durchmesser 0,45 µm) filtrieren;

Die Endkonzentration 3-Amino-1,2,4-Triazol im Medium variiert je nach Experiment zwischen 0 und 50 mM.

Standardmäßig werden Omnitray-Platten (Firma Nunc), auf denen die Hefen für das Scannen im LSA-Scanner kultiviert werden, mit einem Volumen von 78 ml Medium gegossen. Hierdurch entsteht immer eine gleiche Scanhöhe für den Scanner, die auf 9,9 mm eingestellt ist.

### (ii) Durchführung der Hefe-Transformation

Folgendes auf die höchstmögliche Transformationseffizienz optimierte Protokoll kommt zur Anwendung.

20-30 ml flüssiges YPAD (Vollmedium) werden mit den zu transformierenden Hefen (Y190D mit integriertem Reportergen) beimpft und über Nacht bei 30°C und 200 rpm inkubiert. Am nächsten Tag werden zu 50 ml YPAD (auf Raumtemperatur aufgewärmt) Hefen von der Vorkultur hinzupipettiert bis eine OD₆₀₀ von etwa 0,05 erreicht ist. Die Kultur wird bei 30°C und 150-200 rpm inkubiert, bis eine Zelldichte von 2x10⁶ - 4x10⁶ Zellen/ml erreicht ist. Dieses entspricht einer OD₆₀₀ von 0,2 - 0,4 (dauert ca. 3-5 h). Die Kultur wird in einem sterilen 50 ml Zentrifugen-Röhrchen bei 3000 x g (3500 rpm in der Hettich-Zentrifuge) und 5 Minuten geerntet. Das Medium (Überstand) wird entfernt und die Zellen werden in 25 ml sterilem ddH₂O resuspendiert. Nach dem Resuspendieren wird erneut 5 Minuten bei 3000 x g zentrifugiert (3500 rpm Hettich-Zentrifuge). Der Überstand wird entfernt und die Zellen werden in 1,0 ml 100 mM LiAc resuspendiert. Die Suspension wird in ein 1,5 ml Eppendorfcup überführt. Nun werden die Zellen 15 Minuten bei 30°C inkubiert. Anschließend werden die Zellen durch Zentrifugation "full speed" für 15 Sekunden pelletiert und der Überstand abpipettiert. Diese Menge an Zellen reicht für einen Transformationsansatz. Wenn 2 Transformationsansätze gemacht werden sollen, müssen 100 ml (2 x 50 ml) kompetente Zellen hergestellt werden und mit Lithiumacetat vorbehandelt werden. Zu den Zellen wird der folgende "Transformationsmix" in der angegebenen Reihenfolge pipettiert:
X µl Plasmid DNA (0,1-10 µg)
34-X µl Steriles ddH2O
(Zellen in Wasser + Plasmid-Lösung resuspendieren durch Auf- und Abpipettieren, dann erst PEG dazu pipettieren)
240 µl PEG (50 % w/v)
(Zellen mischen mit PEG durch kurzes Vortexen)
36 µl 1,0 M LiAc
50 µl ss-DNA (2,0 mg/ml)
360 µl totales Volumen

Die Zellen werden kräftig gevortext, bis eine homogene Suspension entstanden ist (ca. 1 min). Der Transformationsansatz wird bei 30°C 30 min im Schüttler (800 rpm) inkubiert und anschließend bei 42°C ins Wasserbad gestellt (Heat-Schock). Nach den Inkubationen wird der Transformationsansatz 15 Sekunden bei 6-8000 rpm zentrifugiert und der Transformationsmix mit einer Eppendorfpipette aus dem Eppendorfcup entfernt. Zu dem Pellet (Zellen) wird 1,0 ml steriles ddH₂O gegeben und das Pellet wird durch langsames Auf- und Abpipettieren resuspendiert. Schnelles Auf- und Abpipettieren verringert die Transformationseffizienz. Die aufgelösten transformierten Zellen werden einmal 1:100 und einmal 1:10.000 verdünnt und zwischen 2 und 200 µl der verdünnten Zellen werden auf SC-Medium ohne Leucin, Tryptophan und Histidin mit einer geeigneten Konzentration an 3-Aminotriazole ausgestrichen. Die Anzahl der zu erwartenden Kolonien liegt bei einem ausplattierten Volumen von 20 - 200 µl von einer Verdünnung von 1:10.000 bei 0 - 50 Kolonien pro Platte und bei einem ausplattierten Volumen von 10 - 200 µl von einer Verdünnung von 1:100 bei 200 - > 5000 Kolonien pro Platte. Die Transformationseffizienz liegt dann bei 500.000 - 2.000.000 Zellen pro µg Plasmid-DNA (bei steigender 3-AT Konzentration nimmt die Transformationseffizienz ab). Die Platten werden 2 - 6 Tage bei 30°C bebrütet.

Nach 2 - 6 Tagen (je nach Interaktionspaar und 3-AT-Konzentration) können die Hefezellen Im Scanner LS-400 (Tecan) gescannt und ausgewertet werden.

### (iii) Scannen mit dem Tecan LS-400

Die Hardware (der Scanner Tecan LS-400) mit passender Software ist bei Tecan erhältlich.
Die Höhe des Agars (Scanhöhe) der Omnitray Platte beträgt mindestens 8,0 mm.
Es wurden für die Messungen fertige Methoden angelegt. Klone mit Redstar, cobA oder Met1 im Genom und Two-Hybrid- oder n-Hybrid-Plasmiden werden mit dem
Laser 543 nm und dem Filter 590 nm (Bandpass 20 nm) gescannt.
Die Aufnahmen werden nicht-konfokal durchgeführt.
Scan Resolution (Bildauflösung) ist beim Scannen einer normal gewachsenen Kultur (Durchmesser ca. 1-2 mm) auf 20 µm eingestellt. Wenn eine Kultur aus kleineren Kolonien besteht, wird die Scan Resolution auf 4 bis 8 µm herunter gesetzt.

### (iv) Auswertung der gescannten Kolonien mit Optimate

Zum Auswerten von der Messungen wird die Software Optimate benutzt, die für diese Anwendung in Kooperation entwickelt wurde und kommerziell bei Tecan erhältiich ist.
Folgende Einstellungen sind optimiert für eine Kultur, die aus Kolonien mit einem Durchmesser von 1-2 mm besteht:
Minimum Object: 70
Roundness: 15.2
Threshold Power: 15

Alle Kolonien, die einzeln liegen und groß genug sind, werden ausgewertet. Die Fluoreszenzintensität wird auf die Fläche normiert.

### c) Klonierung des Kompetitors

### (i) Wahl verschiedener Promotoren für den Kompetitor - Vortest

Die Konzentration des Kompetitors ist wesentlich für das n-Hybrid System. Je mehr Genprodukt vorhanden ist, desto mehr verschiebt sich das Gleichgewicht auf die Seite des inaktiven Komplexes aus Kompetitor und Fusions-Köderprotein. Um variable Konzentrationen des Kompetitors zu gewährleisten, sollten verschiedene, in der Literatur (Nacken et al, 1996) als konstitutiv beschriebene Promotoren zur Expression des Kompetitors eingesetzt und in dem Hefestamm validiert werden.
KEX2 (Fuller et al. 1989, M24201), 488 bp
KEX2-for: ATCCTTGAGCTCTCAGCAGCTCTGATGTAGATACAC (Sacl)
KEX2-rev:
ATCCCCCATGGCTGATAATGGGTTAGTAGTTTATAATTATGTG (Ncol)
TEF (Cottrelle et al., 1985, M10992) 411 bp
TEF-for: ATCCCCGCGGTAGCTTCAAAATGTTTCTACTCC (SacII)
TEF-rev: ATCCCCCATGGTTTGTAATTAAAACTTAGATTAGATTG (Ncol)
GAPDH (Bitter und Egan, 1984, M13807), 13, 680 bp
GAPDH-for: ATCCCCGCGGCAGTTCGAGTTTATCATTATCAATAC (SacII)
GAPDH-rev: ATCCCCCATGGTTTGTTTGTTTATGTGTGTTTATTC (NcoI)

Diese Promotoren wurden mit den angegebenen Primem aus dem Hefe-Genom amplifiziert (SacII bzw. SacI/NcoI), mit dem RedStar-Gen (BspHI/NotI) zusammen in pRS306 (SacII bzw. SacI/NotI) kloniert und in das Hefegenom integriert.

Alle drei Promotoren wurden im Folgenden vor den Kompetitor kloniert (s. folgenden Abschnitt).

### (ii) Klonierung des Beuteprotein-Kompetitors auf das Fusions-Köderprotein-Plasmid

Für das erfindungsgemäße Verfahren wird der Kompetitor in eines der beiden Two-Hybrid-Plasmide kloniert und so idealerweise, wie das Köder- und Beuteprotein, von der Zelle selbst synthetisiert. Soll das Beuteprotein als Kompetitor verwendet werden, wird dieses auf den Vektor mit dem Fusions-Köderprotein kloniert; soll das Köderprotein als Kompetitor vorliegen, wird dieses auf den Vektor mit dem Fusions-Beuteprotein kloniert. Hierdurch werden mögliche Rekombinationen zwischen gleichen Gensequenzen, die in der Hefe stattfinden können, vermieden.
Als Ausführungsbeispiel soll die Klonierung von RafRBD (Beuteprotein-Kompetitor) auf pPC97-ras (Fusions-Köderprotein-Plasmid) beschrieben werden.

In pPC97-ras liegt folgende Struktur vor:
Promotor (ADH) - GAL4-BD - ras - mcs (AatII/SacI/SacII) - Terminator (ADH)
Um den Kompetitor einklonieren zu können, muss ein Terminator nach dem ras-Gen eingefügt werden; dann soll der Promotor und dann der Kompetitor folgen. Hierzu wird der Terminator verwendet, der auch an sonstige zu klonierende Gene angehängt wird (s. Amtz et al., 2004). In diesem Fall werden zwei Oligos annealt (Term-Raf-for und -rev; Sequenz s.u.). Hierzu werden die Oligos in einer Endkonzentration von je 2 pmol/µl in einer PCR-Maschine (94°C 2 min, 70 x -1°C, jeweils 1 min bei dieser Temperatur, 4°C; Angabe der Firma Pierce: Anneal complementary pairs of oligonucleotides, Technical Resource) annealt. Für die anschließende Ligation in den Vektor werden 2 µl eingesetzt.

Die Klonierung in das Fusions-Köderprotein-Plasmid erfolgt über AatII/SacI. Das RafRBD-Gen wird amplifiziert (PciI/SacII) und zusammen mit dem jeweiligen Promotor der Wahl (SacI/NcoI) in einer Drei-Fragmente Ligation in den Vektor mit Terminator kloniert (SacI/SacII). Es ergibt sich die aus Abb. 11a ersichtliche Struktur.

Verwendete Primer:
Term-Raf-for: CTATATAACTCTGTAGAAATAAAGAGTATCATCTTTCAAAGAGCT
Term-Raf-rev:
   CTTTGAAAGATGATACTCTTTATTTCTACAGAGTTATATAGACGT
RafRBD-Pci-for: AATTCCACATGTCCGACCCGAGTAAGACAAGC (Pcil)
RafRBD-SacII-rev:
   ATTGCCGCGGTTAGTCGACATCTAGAAAATCTACTTGAAG (Sacll)

### 5. Durchführung des robotergestützten Hitpickings

### (i) Hitpicking mit dem Tecan Freedom 200

Die Hard- und Software sind bei der Firma Tecan kommerziell erhältlich; dabei wurde die Software in Kooperation entwickelt.

In dem Programm Gemini wird das Skript "Colony-Pick" ausgeführt. Dabei wird eingegeben, wie viel % Hits gepickt werden sollen. 70 % Ethanol wird im Behälter "Steril 1" für die Sterilisierung der Pipetier- und Pick-Nadeln bereitgestellt. Die gepickten Kolonien werden in Mikrotiterplatten abgesetzt, die das gleiche Selektionsmedium (SC-LWH-Agar für selektionierte Hefekultivierung im Two-Hybrid und n-Hybrid) enthalten wie das, auf dem die zu pickenden Hefen kultiviert wurden.

In der Software Facts wird der Prozess ColonyPicking ausgeführt; hier kann eine Methode ausgewählt werden, wie gescannt werden muss. Für Colony-Pick (Gemini) ist die Methode festgelegt. Und zwar muss für Klone mit dem Redstar- oder dem cobA-Gen als Reportergen die Methode "Redstar-Scanning" mit folgenden Einstellungen ausgeführt werden:
Scan Area: Top 73 mm, Left 2 mm, Bottom 2 mm, Right 114 mm
Autofocus: Z-Scan End 1600 µm, Z-Scan Start 1600 µm
Focus Offset: 0µm, Focal Plane: Plane 1
Laser: 543 nm, Filter: 590 nm, Scan Resolution: 20 µm, Pinhole: Large

Die Omnitray-Platten müssen mit einem Barcode versehen werden.
Nachdem die Kolonien gepickt worden sind und 2 Tage im Brutschrank bei 30°C kultiviert wurden, werden die Plasmide wieder aus den Hefen isoliert und nach einer Transformation in Bakterien sequenziert.

### (ii) DNA-Isolation aus Hefen mit dem T-Mags von Tecan

Nachdem die Hefen 2 Tage in den Mikrotiterplatten kultiviert wurden, kann nun die DNA-Isolierung aus den Hefen durchgeführt werden. In eine Deepwell-Platte werden in jedes Well 1000 µl Medium (SC-LWH) pipettiert. Nun werden die Kolonien von der Resource-Platte (Platte, auf der die Hefe-Kolonien nach dem Picken wachsen) überimpft in die Deepwell-Platte mit dem jeweiligen Selektionsmedium. Es wird zu den Hefen in der Mikrotiterplatte etwa 200 µl Medium (SC-LWH)/Well pipettiert und durch mehrmaliges Auf- und Ab- pipettieren resuspendiert. Anschließend werden die resuspendierten Hefen überführt in die Deepwell-Platte, in der vorher schon Medium vorgelegt wurde. Nun werden die Hefen etwa 16 Stunden bei 30°C auf einem Mikrotiterplattenschüttler inkubiert.

Am nächsten Tag wird von einigen Wells die optische Dichte bestimmt, indem 100 µl dieser Zellen aus einem Well zu 900 µl Medium gegeben werden. Von dieser 10-fach Verdünnung wird dann die optische Dichte bestimmt. Hiermit bestimmt man dann den Mittelwert von allen Wells. Die Deepwell-Platte mit den Zellen wird dann bei 2500 rpm, 5 Minuten im Ausschwingrotor zentrifugiert (Sorvall Zentrifuge). Als Gegengewicht wird eine andere Deepwell-platte mit der gleichen Volumen mit H₂O befüllt. Nach dem Zentrifugieren wird durch Dekantieren der Deepwellplatte der Überstand entfernt. In jedes Well werden 300 µl Y1-Puffer pipettiert. Zu jedem Well werden außerdem 1-2 Units Lyticase/OD₆₀₀ (der Hefen in den Wells) pipettiert. Puffer und Lyticase werden gut mit den Zellen vermischt. Die Deepwell-Platte wird 1,5 Stunden bei 30°C inkubiert (Nicht schütteln). Die Deepwell-Platte wird bei 2500 rpm, 5 Minuten im Ausschwingrotor zentrifugiert (Sorvall Zentrifuge). Nach dem Zentrifugieren wird durch Dekantieren der Deepwellplatte der Überstand entfernt. Die Zellen in der Deepwell-Platte werden in 250 µl ddH₂O aufgenommen. Die Zellen müssen gut resuspendiert werden. Die Deepwell-Platte ist damit fertig für die DNA-Isolierung.

Mit der Software Gemini des Tecan-Roboters wird eine Methode zur Isolation der DNA ausgeführt, die von der Firma AGOWA (Berlin) zusammen mit Tecan entwickelt worden ist. Diese Isolation findet in dem Gerät T-Mags auf der Roboterplattform statt.

### (iii) Transformation von Bakterien

### Herstellung kompetenter Bakterien zur Transformation in PCR-Platten

5 ml SOB-Medium werden mit einer Einzelkolonie *E.coli*-DH5alpha-Bakterien angeimpft. Die Zellen werden über Nacht bei 37°C im Schüttler (210-225 rpm) inkubiert. 50-100 µl dieser Kultur werden in 100 ml SOB-Medium überführt und bei 37°C im Schüttler (180 rpm) inkubiert. Bei OD₆₀₀ = 0,1 bis 0,5 (nach ca. 2-3 Stunden) werden die Bakterien geerntet und für 20 min auf Eis gestellt. Ab hier erfolgen alle weiteren Schritte bei einer Temperatur von 4°C. Die Bakterienkultur wird im 50 ml Falcongefäß (Spitzboden) bei 4°C und 1200 g zentrifugiert. Das Pellet wird in 10 ml eiskalter 50 mM CaCl2-Lösung durch Auf- und Abpipettieren resuspendiert und anschließend für mindestens 30 min auf Eis inkubiert. Nun werden die Zellen bei 4°C und 1200 g 5 min zentrifugiert. Die Zellen werden in 1 ml/0,1 OD₆₀₀ eiskalter 50 mM CaCl2-Lösung mit 15 % Glycerin durch Auf- und Abpipettieren resuspendiert. Bei einer OD₆₀₀ = 0,1 werden die Zellen also in 1 ml, bei einer OD₆₀₀ = 0,3 in 3 ml CaCl2 aufgenommen. 10 µl-Aliquots pro Well werden in eine auf Eis vorgekühlte PCR-Platte gefüllt und bei - 80°C eingefroren und gelagert.

### Transformation der Bakterien mit der DNA aus Hefen

Das Auftauen der kompetenten Bakterien in den PCR-Platten erfolgt in einem PCR-Block aus Metall, der auf Eis steht. Zu jedem Well werden 10 µl isolierte DNA hinzupipettiert. Bakterien und DNA werden vorsichtig gemischt (nicht Auf- und Abpipettieren!). Die Zellen werden nun mindestens 30 min auf Eis inkubiert. Anschließend wird ein Hitzeschock von 30 s auf einem Heizblock bei 42°C ausgeführt. Nach dem Hitzeschock kommen die Zellen erneut für 2 min auf Eis. In einer Deepwell-Platte werden 100 µl SOC Medium vorgelegt. Zu den Bakterien werden auch 100 µl SOC pro Well hinzupipettiert. Die Bakterien werden von der PCR-Platte in die Deepwell-Platte überführt und für 1 Stunde bei 37°C in dem Mikrotiterplattenschüttler bei 210 bis 225 rpm inkubiert. Nach 1 Stunde Inkubation wird 1 ml LB-Medium mit entsprechendem Antibiotikum zu den Zellen hinzupipettiert und für mindestens 20 Stunden bei 37°C in dem Mikrotiterplattenschüttler bei 210 bis 225 rpm inkubiert. Nach dieser Inkubation werden 5-10 µl der Zellen auf eine 96-Well Platte mit LB-Agar + Antibiotikum überführt und diese Platte wird 16 Stunden bei 37°C im Brutschrank inkubiert. Diese Platte kann zur Sequenzierung der einzelnen Kolonien zu AGOWA geschickt werden.

### 6. Zufallsmutagenese

### Mutagenese auf RafRBD A85

### (i) Konstruktion der notwendigen Vektoren

Nach der Transformation der mutagenisierten Bank befinden sich zwei Plasmide in der Hefe, die beide ein Ampicillin-Resistenzgen tragen. Zum Einen pPC97 mit dem ras-Gen (im n-Hybrid-System befindet sich auf diesem Plasmid noch zusätzlich der Kompetitor; s. Abb. 11) und zum Anderen pPC86, von dem das mutierte RafRBD-Gen codiert wird. Nach der Transformation der aus diesen Hefen isolierten DNA in kompetente Bakterien soll nur noch das Plasmid, auf dem das mutierte RafRBD-Gen vorhanden ist, in den Bakterien vorliegen. Zu diesem Zweck muss einer der Vektoren mit einer anderen Antibiotikumresistenz ausgestatten werden. Im vorliegenden Fall wurde pPC86 mit einer Kanamycinresistenz versehen. Hierzu wurde vor und nach dem TEM-Resistenzgen mittels QuikChange Mutation nach Angaben des Herstellers jeweils eine Pmel-site generiert, das Gen danach ausgeschnitten und durch das Kanamycin-Resistenzgen ersetzt. Die mit der DNA aus Hefe transformierten Bakterien wachsen nun in Medium mit Kanamycin und können so von den Bakterien, die das Fusions-Köderprotein-Plasmid pPC97 enthalten, separiert werden.

Verwendete Primer:
Multi-QC-Pme-vor-TEM
   TGAATACTCATACTCTTCCTGTTTAAACATTATTGAAGCATTTATCAGGG
Multi-QC-Pme-nach-TEM:
   TTAAATCAATCTAAAGTATATATGTTTAAACTTGGTCTGACAGTTACCAATG (PmeI)
Pme-Kan-for:
   AAAAAACCGTTTAAACAGGAAGAGTATGATTGAACAAGATGGATTGC (Pmel)
Pme-Kan-rev:
   AAAAAACCGTTTAAACTTGGTCTGACAGTCAGAAGAACTCGTCAAGAAGG (Pmel)

Die Zufallsmutagenese wird nach der Methode von Zheng et al. (2004) durchgeführt (s. Abschnitt b). Hierzu wurden folgende zwei Primer entworfen, die teilweise überlappen und die Aminosäure A85 von RafRBD randomisieren:
Z-RBO-A85-forl: CTGCCTTATGAAANNKCTCAAGGTGAGGGGCCTGCAACCAG
Z-RBD-A85-rev CCCTCACCTTGAGMNNTTTCATAAGGCAGTCATGCAAGCTC

Diese Primer werden zu einer PCR mit dem Expand Kit (Roche) eingesetzt. Hierfür werden 50 ng Template-DNA (pPC86-RafRBD mit Kanamycin-Resistenzgen) verwendet und die PCR nach Angaben des Herstellers mit 0,8 pmol/µl jedes Primers durchgeführt. Die PCR-Reaktion wird dann mit dem PCR Purification Kit (Qiagen) aufgereinigt und 5 von 50 µl werden auf ein Agarosegel aufgetragen. Der restliche Ansatz wird mit 10 U Dpnl (NEB, in Puffer 4) 3 Stunden bei 37°C restringiert, um die methylierte, da aus *E.coli* isolierte, Template-DNA zu entfernen. Zheng et al. führten hier den Verdau nur 1 Stunde durch, was aber in einem hohen Hintergrund an Wildtyp-Klonen in der Bank resultierte.

Transformiert werden dann 2,5 µl des Dpnl-Cuts in 75 µl kompetente XL10 Gold-Zellen (Stratagene) nach Angaben des Herstellers; nach Zugabe von 750 µl NZY nach dem Hitzeschock erfolgt eine einstündige Regenerationsphase der Bakterien. 20 µl des Transformationsansatzes werden zur Bestimmung der Transformationseffizienz ausplattiert (ca. 1/20 des Gesamtansatzes) und der Rest wird in LB-Medium mit Kanamycin über Nacht bei 37°C und 225 rpm auf dem Schüttler inkubiert. Am nächsten Morgen wird die DNA isoliert. Die Bestimmung der Anzahl an voraussichtlich unabhängigen Kolonien erfolgt durch Zählen der ausplattierten Kolonien und Hochrechnen auf die Gesamtzahl (Faktor 20).

Die Bank wird durch Sequenzierung von Einzelkolonien und der Bank-DNA charakterisiert. Diese DNA wird dann in die Hefe transformiert, die ein Reportergen im Genom enthält. Dabei wird einmal pPC97-ras (für das Two-Hybrid) und einmal pPC97-ras mit Kompetitor TEF-Promotor/RafRBD (für das n-Hybrid System,) als zweites Plasmid eingesetzt.

### Literatur:

Amtz, C., Meinders, D., Block, C., Mittmann, K. (2004) Phycocyanin exprimierende Eukaryontenzelle. EPA 04 001 504.2
Bitter GA and Egan KM (1984) Expression of heterologous genes in Saccharomyces cerevisiae from vectors utilizing the glyceraldehydes-3-phosphate dehydrogenase gene promoter. Gene 32:263-274
Block C., Janknecht R., Herrmann C., Nassar N., Wittinghofer A. (1996) Quantitative structure-activity analysis correlating Ras/Raf interaction in vitro to Raf activation in vivo. Nature Structural Biology 3:244-251
Boeke JD, LaCroute F, Fink GR. (1984) A positive selection for mutants lacking orotidine-5'-phosphate decarboxylase activity in yeast: 5-fluoro-orotic acid resistance. Mol. Gen. Genet. 197(2):345-6
Bosley AD, Ostermeier M (2005) Mathematical expressions useful in the construction, description and evaluation of protein libraries. Biomolecular Engineering 22:57-61
Botstein D, Falco S et al. (1979) Sterile host yeasts (SHY): A eukaryotic system of biological containment for recombinant DNA experiments. Gene 8:17-24
Cottrelle P, Thiele D, Price V, Memet S, Micouin J-Y, Marck C, Buhler J-M, Sentenac A, Fromageot P (1985) Cloning, Nucleotide Sequence, and Expression of One of Two Genes Coding for Yeast Elonation Factor 1a. JBC 260:3090-3096
Fridman, M., Maruta, H., Gonez, J., Walker, F., Treutlein, H., Zeng, J. und Burgess, A. (2000) Point Mutants of c-raf-1 RBD with elevated Binding to v-Ha-Ras. J. Biol. Chem. 275, 30363-30371
Fusco C, Guidotti E, Zervos AS (1999) In vivo Construction of cDNA Libraries for Use in the Yeast Two-Hybrid System. Yeast 15:715-720
Fuller RS, Brake A, Thomer J (1989) Yeast prohormone processing enzyme (KEX2 gene product) is a Ca 2+-dependent serine protease. PNAS 86:1434-1438
Guarante L. Ptashne M (1981) Fusion of Escherichia coli lacZ to the cytochrome c gene of Saccharomyces cerevisiae. PNAS 78:2199-2203
Hogrefe HH, Cline J, Youngblood GL, Allen RM (2002) Creating Randomized Amino Acid Libraries with the QuikChange Multi Site-Directed Mutagenesis Kit. BioTechniques 33(5):1158-1165
Hua S, Luo Y, Qiu M, Chan E, Zhou H, Zhu L (1998) Construction of a modular yeast two-hybrid cDNA library from human EST clones for the human genome protein linkage map. Gene 215:143-152
Jaitner, B.K., Becker, J., Linnemann, T., Herrmann, C., Wittinghofer, A. und Block C. (1997) Discrimination of Amino Acids mediating Ras binding from noninteracting Residues affecting Raf Activation by Double Mutant Analysis. J. Biol. Chem. 272, 29927-29933
Knop, M., Barr, F., Riedel, CG., Reichel, C. (2002) Improved Version of the Red Fluorescent Protein (drFP583/DsRed/RFP). Bio Techniques 33:592-602
Leuther KK, Jonston SA (1992) Nondissociation of GAL 4 and GAL 80 in Vivo After Galactose Induction. Science 256:1333-1335
Muhlrad D, Hunter R, Parker R (1992) A Rapid Method for Lacalized Mutgenesis of Yeast Genes. Yeast 8:79-82
Nacken V, Achstetter T, Degryse E (1996) Probing the limits of expression levels by varying promoter strength and plasmid copy numer in Saccharomydes cerevisiae. Gene 175:2563-260
Palfrey D, Picardo M, Hine AV (2000) A new randomization assay reveals unexpected elements of sequence bias in model "randomized" gene libraries: implications for biopanning. Gene 251:91-99
Roessner C.A. (2002) Use of cobA and cysGA as red fluorescent indicators. Methods Mol. Biol. 183, 19-30
Schaerer-Brodbeck C, Barberis A (2004) Coupling homologour recombination with growth selection in yeast: a tool for construction of random DNA sequence libraries. BioTechniques 37:202-206
Sneeden JL, Loeb LA (2003) Random Oligonucleotide Mutagenesis. In: Methods in Molecular Biology, Vol. 231:65-73. Edited by FH Amold and G. Georgiou. Humana Press
Stemmer WPC (1994) DNA shuffling by random fragmentation and reassembly: In vitro recombination for molecular evolution. PNAS 91:10747-10751
Treco DA Basic Techniques of Yeast Genetics (1989) In: Current Protocols in Molecular Blology, Wiley, Supplement 5, Unit 13.1, p 13.1.5
West RW, Yocum RR, Ptashne M. (1984) Saccharomyces cerevisiae GAL1-GAL10 Divergent Promoter Region: Location and Function of the Upstream Activating Sequence UASG Mol Cell Biol Nov. 1984:2467-2478
Wildt S and Deuschle U (1999) cobA, a red fluorescent transcriptional reporter for Escherichia coli, yeast, and mammalian cells. Nat Biotech 17:1175-1178
Yocum RR, Hanley S, West R, Ptashne M (1984) Use of IacZ Fusions to Delimit Regulatory Elements of the Inducible Divergent GAL1-GAL10 Promoter in Saccharomyces cerevisiae. Mol Cell Biol Oct. 1984:1985-1998
Zheng L, Baumann U, Reymond J-L (2004) An efficient one-step site-directed and site-saturation mutagenensis protocol. Nucleic Acids Research 32(14): e115

### Annex zu den Anmeldungsunterlagen - nachgereichtes Sequenzprotokoll

## Patentansprüche

1. Verfahren zur Erzeugung von genetischer Diversität *in vivo* umfassend folgende Schritte:
- Erzeugen von Fragmenten mindestens einer Wildtyp-Gensequenz, wobei die Fragmente jeweils mindestens einen zu einem für die *in vivo* Rekombination geeigneten Vektor homologen Bereich aufweisen;
- Erzeugen von randomisierten Überbrückungsoligonukleotiden mindestens einer definierten Oigonukleotidseqenz, wobei Teile des Überbrückungsoligonukleotids mit mindestens einem Fragment der Wildtyp-Gensequenz homolog sind und
- Einbringen des linearisierten Vektors, mindestens eines Wildtyp-Fragments sowie des/der randomisierten Überbrückungsoligonukleotide in ein *in vivo* System zur homologen Rekombination.

2. Verfahren nach Anspruch 1, **dadurch gekennzeichnet, daß** die Überbrückungsoligonukleotide über nicht-PCR basierte Verfahren randomisiert werden.

3. Verfahren nach Anspruch 1 oder 2, **dadurch gekennzeichnet, daß** die Wildtyp-Fragmente über ein PCR-basiertes Verfahren oder aber synthetisch erzeugt werden.

4. Verfahren nach Anspruch 3, **dadurch gekennzeichnet, daß** in die Wildtyp-Sequenz ein Wildtyp-fremder DNA-Abschnitt ("Stuffer-Fragment") einkloniert wird.

5. Verfahren nach einem der vorherigen Ansprüche, **dadurch gekennzeichnet, daß** der Wirtsorganismus mit mindestens vier Wildtyp-Fragmenten transformiert wird, wobei nur zwei einen zu dem Vektor homologen Sequenzbereich aufweisen.

6. Verfahren nach einem der vorherigen Ansprüche, **dadurch gekennzeichnet, dass** der Wirtsorganismus mit mehr als einem randomisierten Überbrückungsoligonukleotid transformiert wird.

7. Verfahren nach einem der vorherigen Ansprüche, **dadurch gekennzeichnet, dass** die Transformation in Hefe erfolgt.

8. Verfahren nach Anspruch 7 **gekennzeichnet durch** eine Selektion der transformierten Hefeklone über ein Two Hybrid oder ein n-Hybrid System.
